# EUROPEAN PATENT SPECIFICATION

(11) **EP 3 331 601 B1**
(45) Date of publication and mention of the grant of the patent: **10.03.2021**
(21) Application number: 16832404.4
(22) Date of filing: 03.08.2016
(51) Int. Cl.: A61N 1/36, A61B 5/20, A61B 5/00, A61P 13/10, A61N 1/372, A61N 7/00, A61F 7/00, A61N 5/06

(54) **NEUROMODULATION DEVICE**
NEUROMODULATIONSVORRICHTUNG
DISPOSITIF DE NEUROMODULATION

(30) Priority: 03.08.2015 US 201562200211 P
(43) Date of publication of application: 13.06.2018
(73) Proprietor: Galvani Bioelectronics Limited, Brentford Middlesex TW8 9GS (GB); Duke University, Durham, North Carolina 27705 (US)
(72) Inventor: FAMM, Hans Jakob Kristoffer, Stevenage Hertfordshire SG1 2NY (GB); GRILL, Warren Murray, Durham, NC 27705 (US); HOKANSON, James Arthur, Durham, NC 27705 (US); LANGDALE, Christopher Lawrence, Durham, NC 27705 (US); SRIDHAR, Arun, Stevenage Hertfordshire SG1 2NY (GB)
(74) Representative: Boult Wade Tennant LLP
(86) International application number: PCT/IB2016/054687
(87) International publication number: WO 2017/021909

(56) References cited:
- US-A1- 2003 004 553
- US-A1- 2005 033 374
- US-A1- 2008 071 321
- US-A1- 2011 264 163
- US-A1- 2014 249 595
- US-A1- 2015 148 878
- MEREDITH J. MCGEE ET AL: "Selective co-stimulation of pudendal afferents enhances bladder activation and improves voiding efficiency : Co-Stimulation of Pudendal Afferents Improves Voiding", NEUROUROLOGY AND URODYNAMICS., vol. 33, no. 8, 1 October 2014 (2014-10-01), pages 1272-1278, XP055550305, US ISSN: 0733-2467, DOI: 10.1002/nau.22474
- Meredith J. Mcgee ET AL: "A spinal GABAergic mechanism is necessary for bladder inhibition by pudendal afferent stimulation", American Journal of Physiology: Renal Physiology, vol. 307, no. 8, 15 October 2014 (2014-10-15), pages F921-F930, XP055550256, United States ISSN: 1931-857X, DOI: 10.1152/ajprenal.00330.2014

## Description

### BACKGROUND

Efficient bladder function, mediated by continence and micturition reflexes, is accomplished through coordinated sympathetic, parasympathetic and somatic neural activity [Beckel and Holstege Neurophysiology of the Lower Urinary Tract, in Urinary Tract (2011) Springer Berlin Heidelberg, 149-169]].

Treatments for bladder dysfunction include behavioural therapy, exercise therapy, and pharmacotherapy. Behavioural and exercise therapy have limited efficacy, and pharmacotherapy has dose-limiting side effects. Overactive bladder (OAB), resulting in urgency, frequency and incontinence, is a highly prevalent condition that leads to medical complications and decreased quality of life [Latini & Giannantoni (2011), Expert Opinion on Pharmacotherapy 12:1017-1027].

Parasympathetic control of the urinary bladder originates from the sacral spinal cord segments S2-S4. Mechanoreceptors in the bladder wall supply visceral afferent information to the spinal cord and higher autonomic centres in the brainstem. Efferent innervation is supplied to the visceral motor neurons in parasympathetic ganglia in or near the bladder wall. The bladder receives sympathetic innervation from the T10-L2 region of the spinal cord via postganglionic fibres travelling in the hypogastric and pelvic nerves to the bladder. Sympathetic activity causes the internal urethra to close and inhibits contraction of the detrusor. Filling of the bladder increases parasympathetic tone and decreases sympathetic activity, ultimately causing the internal urethral sphincter to relax and the detrusor to contract [Purves et al., Neuroscience 2nd Ed. (2001) Sinauer Associates].

In patients who are non-responsive or whose condition is inadequately controlled by conservative treatments, attempts have been made to control the functioning of the urinary bladder using electrical devices, as summarized by Gaunt and Prochazka (Progress in Brain Research 152:163-94 (2006)). The FDA-approved use of sacral neuromodulation (SNM) targeting the sacral spinal nerves (INTERSTIM™ therapy of Medtronic, Inc (Minneapolis, MN)) has proved partially successful. The Medtronic system uses a cylindrical electrode inserted in the S3 sacral foramen (a bony tunnel in the pelvis) adjacent to the S3 spinal nerve. Approximately half of screened subjects go on to receive an implant, and only around 75% of implant recipients experience a ≥50% reduction in leaking episodes (Schmidt, et al. Sacral nerve stimulation for treatment of refractory urinary urge incontinence, (1999) J Urol. 162(2):352-7). Further, in a multi-centre clinical trial of 98 implanted patients, surgical revision was required in 32.5% of recipients, illustrating the complexity of the spinal nerve approach (Van Voskuilen AC, et al. Medium-term experience of sacral neuromodulation by tined lead implantation. BJU Int 2007;99:107-10; Pham K, et al. Unilateral versus bilateral stage I neuromodulator lead placement for the treatment of refractory voiding dysfunction. Neurourol Urodyn 2008; 27:779-81).

Bladder function is comprised of two phases: a filling phase (urine storage) and a voiding phase (urine evacuation). Despite this biphasic process, artificial electric stimulation protocols do not differentiate between the phases, even though the goals of these phases are diametrically opposed. Instead, it is customary to stimulate with a fixed stimulus amplitude, rate and pulse width throughout the day. Advanced features allow for intervening periods of stimulation and no stimulation (cycling), although this is principally to prolong battery life rather than to specifically target urine storage or voiding (Medtronic INTERSTIM™ Programming Guide), and is not timed with respect to periods of continence (storage) or voiding (micturition).

The Finetech-Brindley bladder control system (Finetech Medical, Hertfordshire, UK) combines cuff-type electrodes implanted on the sacral ventral roots to activate the bladder, combined with surgical transection of the sacral dorsal roots to eliminate hyperactive reflexes. This approach is highly effective in restoring bladder function, but requires a highly invasive surgical procedure and irreversible transection of the sacral sensory nerves, and is limited to persons with complete spinal cord injury.

Others have attempted to modulate the peripheral nerves to control bladder function. Dalmose (Scand J Urol Nephrol Suppl 210: 34-45 (2002)) describes the stimulation of the efferent fibres of the pelvic nerve, prompting bladder contraction. Jezernik et al. J Urol. 163:1309-14 (2000) described electrical recording from the pelvic nerve in pigs as a means to detect changes in bladder pressure.

Grill et al. (US 6,907,293) and Boggs II et al. (US 7,571,000 and US 8,396,555) describe apparatus for stimulating the pudendal nerve or branches thereof, or sacral roots, to control function in the lower urinary tract. Boggs II *et al.* describe an apparatus in which a stimulatory electrode is placed near afferent nerve fibres in the deep perineal nerve and/or a urethral afferent of the pudendal nerve and using modulations in the frequency of a stimulation waveform to inhibit bladder contraction or conversely to evoke contraction.

Rijkhoff et al. (US 6,836,684) describe a method to control OAB in which a recording electrode is positioned on a nerve in a manner to sense the onset of bladder contraction, and a stimulatory electrode is positioned on a nerve in a manner to activate an inhibitory neural circuit. The inhibitory neural circuit is activated by the stimulatory electrode in response to an undesired bladder contraction. The inventors propose that the recorded nerve signal comes from afferent nerve fibres innervating mechanoreceptors in the bladder wall and/or the detrusor, and that the stimulatory electrode activates afferent nerve fibres innervating the glans of the penis or clitoris, which has a strong inhibitory effect on the bladder.

US 2005/033374, US 2008/071321 and US 2015/148878 show apparatuses for stimulating a pelvic nerve. The article by MEREDITH J. MCGEE ET AL, entitled "Selective co-stimulation of pudendal afferents enhances bladder activation and improves voiding efficiency : Co-Stimulation of Pudendal Afferents Improves Voiding" published in Neurourology and Urodynamics (vol. 33, no. 8, 1 October 2014, p. 1272-1278, ISSN: 0733-2467) and the article by Meredith J. Mcgee ET AL entitled "A spinal GABAergic mechanism is necessary for bladder inhibition by pudendal afferent stimulation" published in American Journal of Physiology: Renal Physiology (vol. 307, no. 8, 15 October 2014, pages F921-F930, ISSN: 1931-857X) show apparatuses for stimulation of pudendal nerve afferents.

It would be desirable to provide improved apparatus 1 to provide for control of bladder function.

### SUMMARY OF INVENTION

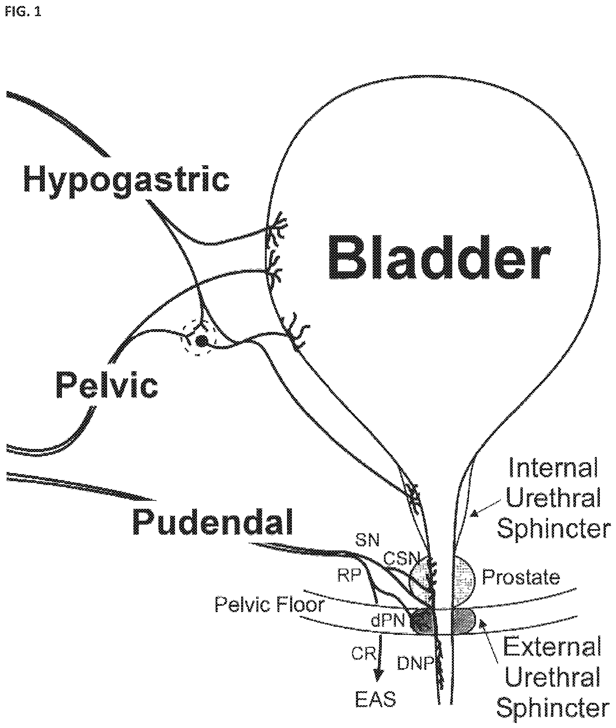

The present inventors have characterised and quantified the dysfunction that results in animal models of overactive bladder, and have identified peripheral nerves (the pelvic and optionally pudendal nerves) innervating the bladder and/or lower urinary tract as targets for treatment of bladder dysfunction. The pelvic nerve is an autonomic (parasympathetic) nerve derived from the sacral spine which innervates the bladder and internal urethral sphincter and carries afferent and efferent nerve signals (Figure 1). The pudendal nerve is a somatic nerve (i.e. not autonomic) that innervates the urethra, external urethral sphincter, external anal sphincter, and perineal skin and carries afferent and efferent signals (Figure 1). Other peripheral nerves innervating the bladder and lower urinary tract include the hypogastric nerve, an autonomic (sympathetic) nerve that innervates the bladder and carries afferent and efferent signals (Figure 1).

The apparatuses provided herein address the problem of treating bladder dysfunction using electrical devices by targeting the afferent fibres of a pelvic nerve and optionally the afferent fibres of a pudendal nerve of the patient. These apparatuses and methods have the advantage of providing greater control of bladder function, whilst not requiring significant and potentially dangerous spinal surgery in order to position devices in signalling contact with these nerves. in addition, the inventors have further found that modulating (optionally increasing) the neural activity in the afferent fibres of at least the pelvic nerve results in the surprising effects of increasing bladder capacity and increasing voiding efficiency., The present inventors have therefore shown that numerous aspects of normal bladder activity can be restored in animal models of abnormal bladder function, thereby showing that such modulation provides effective treatment of bladder dysfunction without the need for acting on spinal nerves. Moreover, selective modulation of (optionally increasing) the neural activity in the afferent fibres of the pelvic nerve is particularly advantageous. Such selective modulation (optionally selective stimulation) of neural activity in the afferent fibres of the pelvic nerve is characterised by the signal modulating (e.g. increasing) neural activity in the afferent fibres, but not modulating neural activity in the efferent nerve fibres of the pelvic nerve to a threshold level at which activity of the external urethral sphincter increases, or urethral pressure decreases. Preferably such selective modulation (e.g. stimulation) does not modulate (e.g. increase) neural signalling in the efferent nerve fibres of the pelvic nerve.

Therefore, the invention provides an apparatus for modulating the neural activity of afferent fibres of at least one pelvic nerve of a patient as defined by claim 1. Preferred embodiments are defined by the dependent claims.

Aspects, embodiments or examples of the present disclosure which do not fall within the scope of the appended claims do not form part of the present invention and are present as background information only.

Methods disclosed hereinafter are also not part of the present invention.

The apparatus comprising: a first actuator configured to apply a first signal to said at least one pelvic nerve of the patient; and a controller coupled to the first actuator, the controller controlling the signal to be applied by the actuator, such that the signal modulates the neural activity of the afferent fibres of said at least one pelvic nerve to produce a physiological response in the patient.

In certain embodiments, the apparatus comprises a second actuator coupled to the controller and configured to apply a second signal to a pudendal nerve of the patient, wherein the controller controls the signal to be applied by the second actuator such that the signal modulates the neural activity of afferent fibres of the pudendal nerve to produce a physiological response in the patient.

In certain embodiments, the modulation is an increase in neural activity of the afferent fibres of the nerve. In certain embodiments the modulation is a selective increase in neural activity of the afferent fibres of the pelvic nerve. That is, in an embodiment, the modulation of neural activity does not increase neural signalling in the efferent nerve fibres of the pelvic nerve, or alternatively, does not increase neural activity in the efferent nerve fibres of the pelvic nerve to a threshold level at which bladder pressure increases.

The present invention can be used in a a method of treating bladder dysfunction, optionally overactive bladder (urgency, frequency and incontinence), neurogenic bladder, stress incontinence, urinary retention, in a patient comprising: implanting in the patient the aforementioned apparatus; positioning the first actuator of the apparatus in signalling contact with at least one pelvic nerve of the patient; and activating the apparatus.

In certain examples, the method further comprises positioning the second actuator of the apparatus in signalling contact with at least one pudendal nerve of the patient.

The present disclosure also relates to a method of treating bladder dysfunction in a patient, the method comprising applying a first signal to at least one pelvic nerve of said patient to modulate the neural activity of afferent nerve fibres of said nerve in the patient.

In certain examples, the method is a method of treating overactive bladder (urgency, frequency and incontinence), neurogenic bladder, stress incontinence, urinary retention. In certain embodiments a second signal is applied to a pudendal nerve of the patient, to modulate the neural activity of afferent nerve fibres of said nerve in the patient. In certain examples, the signal or signals is/are applied by a neuromodulation apparatus comprising at least one actuator configured to apply each signal.

In certain examples, the modulation is an increase in neural activity of the afferent fibres of the nerve. In certain examples, the modulation is a selective increase in neural activity of the afferent fibres of the pelvic nerve. That is, the modulation of neural activity does not increase neural signalling in the efferent nerve fibres of the pelvic nerve, or alternatively, does not increase neural activity in the efferent nerve fibres of the pelvic nerve to a threshold level at which bladder pressure decreases.

The present disclosure also provides a neuromodulatory electrical waveform for use in treating bladder dysfunction in a patient, wherein the waveform is a sub-kilohertz frequency, pulsatile AC waveform having a pulse repetition frequency of 0.5-20 Hz, such that, when applied to a pelvic nerve of the patient, the waveform increases neural signalling in the afferent nerve fibres of the pelvic nerve to which the signal is applied, optionally selectively increases neural signalling in the afferent nerve fibres of the pelvic nerve to which the signal is applied. That is, the waveform does not increase neural signalling in the efferent nerve fibres of the pelvic nerve, or alternatively, does not increase neural activity in the efferent nerve fibres of the pelvic nerve to a threshold level at which bladder pressure increases.

The present disclosure also relates to the use of a neuromodulation apparatus for treating bladder dysfunction in a patient by stimulating neural activity in at least one pelvic nerve of a patient, optionally selectively stimulates neural activity in afferent nerve fibres of the at least one pelvic nerve of the patient.

The present disclosure also provides a neuromodulation system, the system comprising a plurality of the aforementioned apparatuses. I In such a system, each apparatus may be arranged to communicate with at least one other apparatus, optionally all apparatuses in the system. In certain examples, the system is arranged such that, in use, the apparatuses are positioned to bilaterally modulate the neural activity of the afferent fibres of the pelvic nerves of a patient. In certain examples, the system is arranged such that, in use, the apparatuses are positioned to modulate the neural activity of the afferent fibres of at least one pelvic nerve of a patient and to modulate the activity of the afferent fibres of a pudendal nerve of the patient.

The present disclosure also relates to a pharmaceutical composition comprising a compound for treating bladder dysfunction, for use in a method of treating bladder dysfunction in a subject, wherein the method is a method as mentioned above, the method further comprising the step of administering the pharmaceutical composition to the subject.

The present disclosure also relates to a pharmaceutical composition comprising a compound for treating bladder dysfunction, for use in treating bladder dysfunction in a subject, the subject having an apparatus as mentioned above.

In certain examples, the compound for treating bladder dysfunction is an antimuscarinic compound, for example darifenacin, hyoscyamine, oxybutynin, tolterodine, solifenacin, trospium, or fesoterodine. In certain examples, the compound for treating bladder dysfunction is a β-adrenergic receptor agonist, optionally a β3-adrenergic receptor agonist, for example mirabegron. In an alternative examples, the compound is botulinum toxin.

### Figures

- Figure 1:: Schematic drawing showing innervation of the bladder, internal urethral sphincter (IUS), external urethral sphincter (EUS) and prostate. Sensory branch of the pudendal nerve (SN), rectal perineal branch of the pudendal nerve (RP), cranial sensory branch of the pudendal nerve (CSN), dorsal nerve of the penis branch of the pudendal nerve (DNP; or clitoris), deep perineal branch of the pudendal nerve (dPN) and caudal rectal branch of the pudendal nerve (CR).

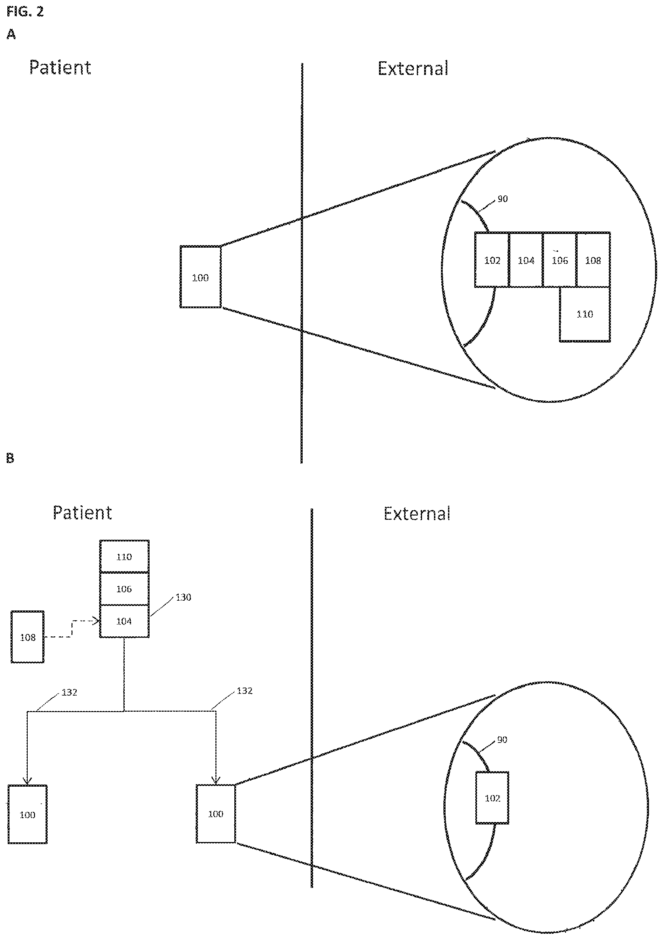

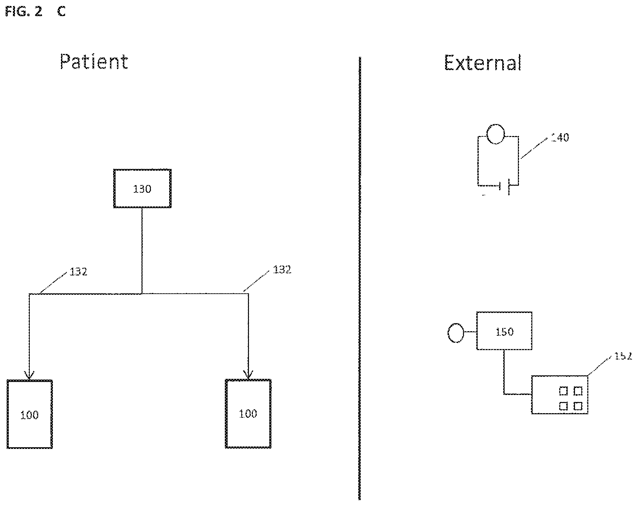

- Figure 2:: Schematic drawings showing how apparatuses, devices and systems can be put into effect.

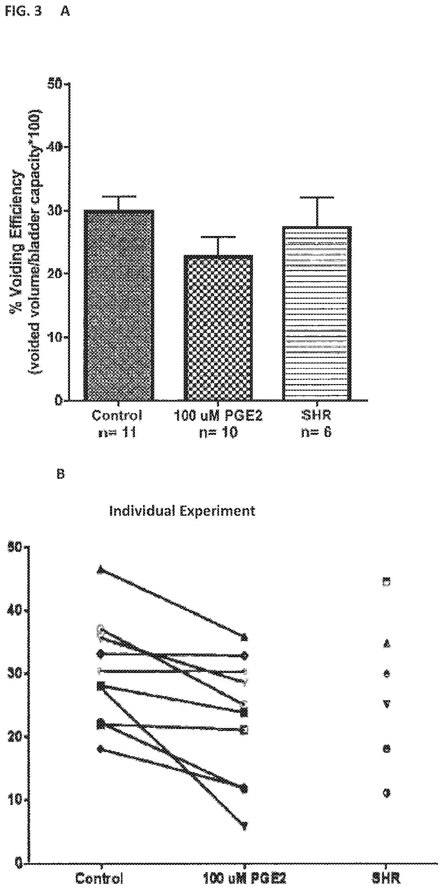

- Figure 3:: A - mean voiding efficiency in PGE2 and SHR models of bladder dysfunction. In this cohort, PGE2 rats exhibited a statistically non-significant reduction in voiding efficiency versus controls. Data is expressed as mean ± SE. N values as indicated. B - Voiding efficiency in individual PGE2 and SHR rats represented in A. Each PGE2 rat prior to PGE2 installation is used as its own control and each data point represents the mean for each experiment.

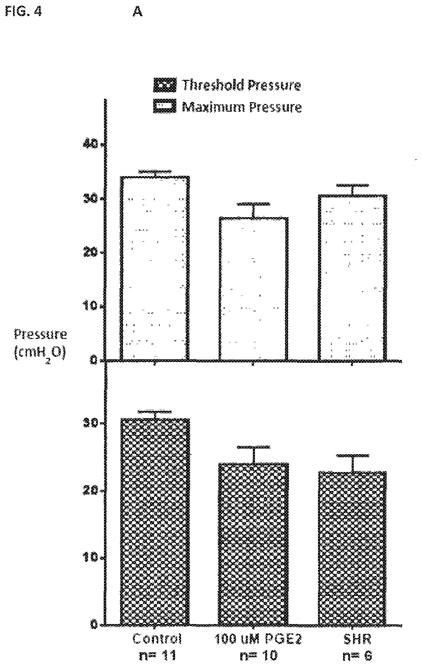

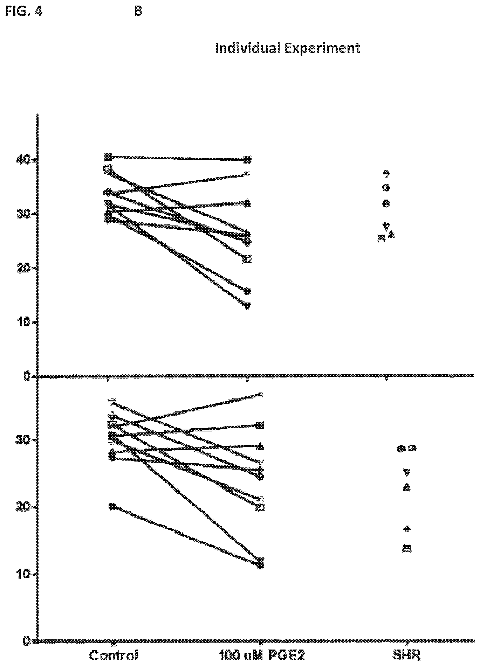

- Figure 4:: A - maximum bladder pressure (top) and threshold bladder pressure (bottom) in PGE2 and SHR models of bladder dysfunction. PGE2 and SHR rats exhibited a reduction in threshold pressure and maximum pressure versus controls. Data is expressed as mean ± SE. N values as indicated. B - Maximum bladder pressure (top) and threshold bladder pressure (bottom)in individual PGE2 and SHR rats represented in A. Each PGE2 rat prior to PGE2 installation is used as its own control and each data point represents the mean for each experiment.

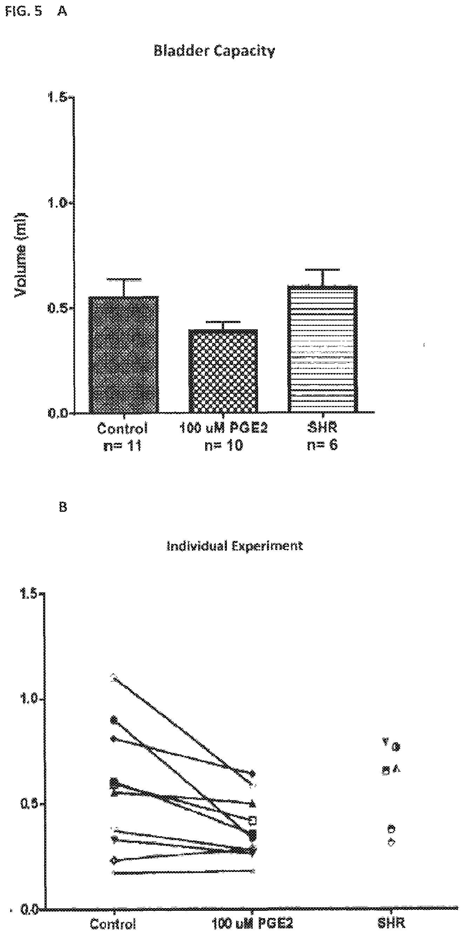

- Figure 5:: A - mean bladder capacity in PGE2 and SHR models of bladder dysfunction. PGE2 rats exhibited a reduction in bladder capacity versus controls. Data is expressed as mean ± SE. N values as indicated. B - Bladder capacity in individual PGE2 and SHR rats represented in A. Each PGE2 rat prior to PGE2 installation is used as its own control and each data point represents the mean for each experiment.

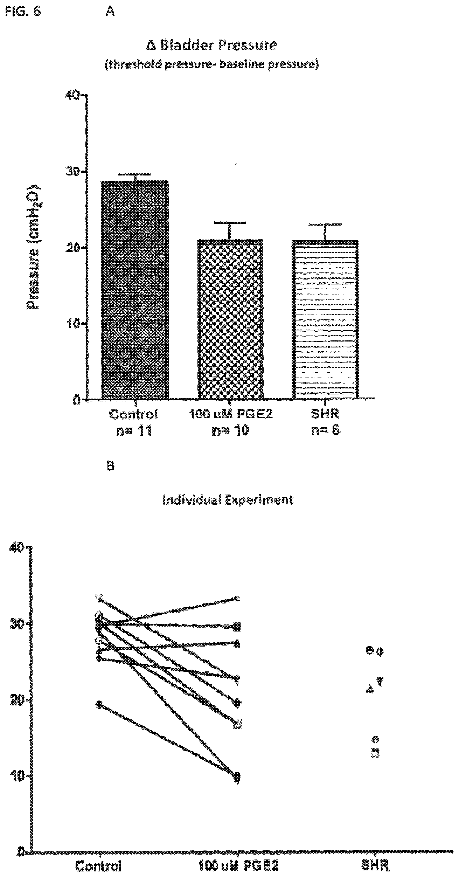

- Figure 6:: A - mean Δbladder pressure in PGE2 and SHR models of bladder dysfunction. PGE2 and SHR rats exhibited a reduction in Δbladder pressure versus controls. Δbladder pressure was calculated by subtracting baseline bladder pressure from the threshold bladder pressure. Data is expressed as mean ± SE. N values as indicated. B - Δbladder pressure in individual PGE2 and SHR rats represented in A. Each PGE2 rat prior to PGE2 installation is used as its own control and each data point represents the mean for each experiment.

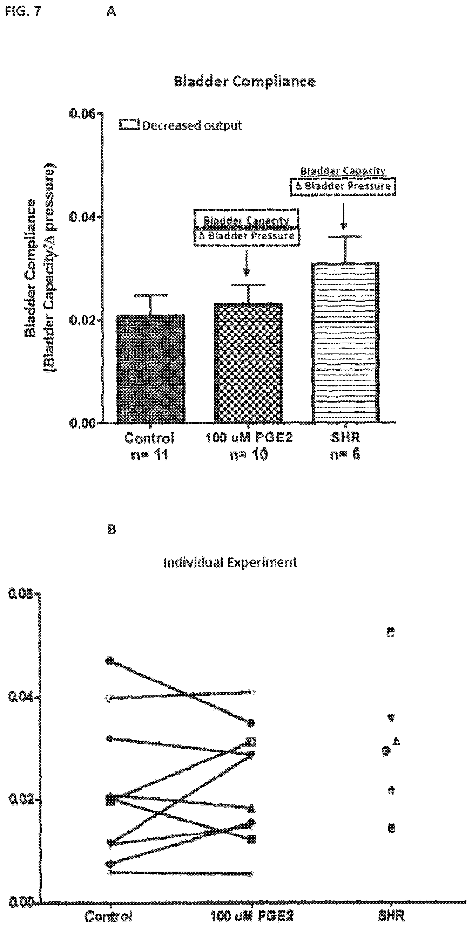

- Figure 7:: A - mean bladder compliance in PGE2 and SHR models of bladder dysfunction. SHR rats exhibited an increase in bladder compliance versus controls. Bladder compliance was calculated by dividing the bladder capacity by the Δbladder pressure . The red box indicates which parameters used to calculate bladder compliance were decreased when compared to control. Data is expressed as mean ± SE. N values as indicated. B - Bladder compliance in individual PGE2 and SHR rats represented in A. Each PGE2 rat prior to PGE2 installation is used as its own control and each data point represents the mean for each experiment.

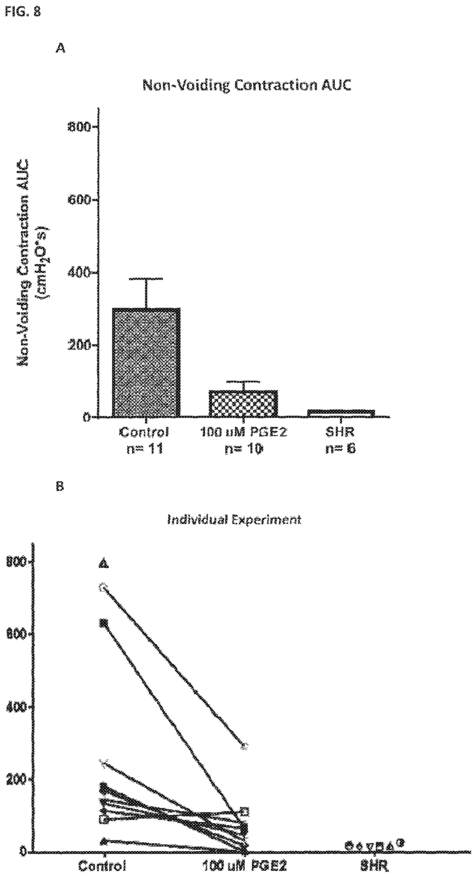

- Figure 8:: A - mean non-voiding contraction (NVC) magnitude (as measured by bladder pressure area under the curve) in PGE2 and SHR models of bladder dysfunction. PGE2 and SHR rats exhibited a reduction in NVC magnitude versus controls. Data is expressed as mean ± SE. N values as indicated. B - NVC magnitude in individual PGE2 and SHR rats represented in A. Each PGE2 rat prior to PGE2 installation is used as its own control and each data point represents the mean for each experiment.

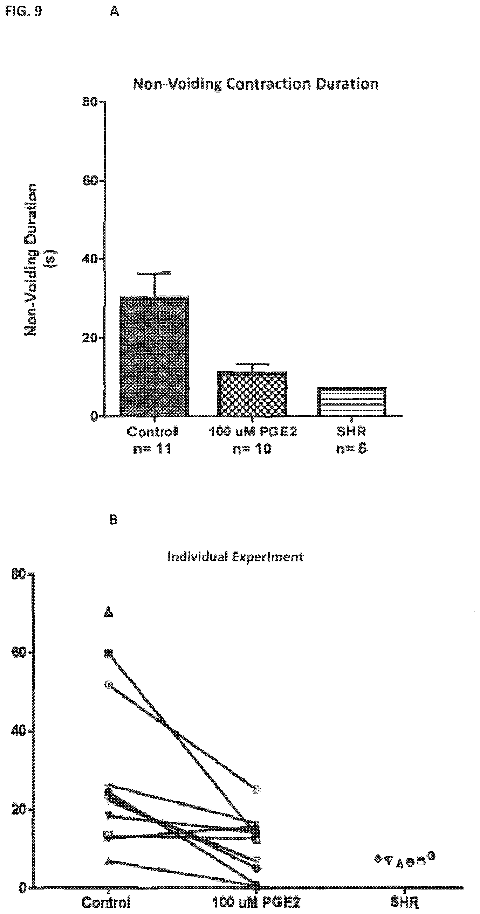

- Figure 9:: A - NVC duration in PGE2 and SHR models of bladder dysfunction. PGE2 and SHR rats exhibited a reduction in NVC duration versus controls. Data is expressed as mean ± SE. N values as indicated. B - NVC duration in individual PGE2 and SHR rats represented in A. Each PGE2 rat prior to PGE2 installation is used as its own control and each data point represents the mean for each experiment.

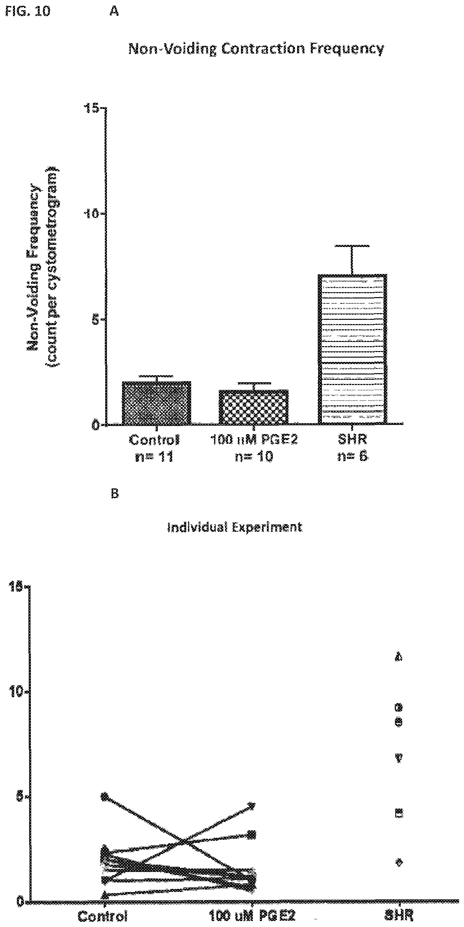

- Figure 10:: A - NVC frequency (as measured by the number of NVC events counted during the filling phase of the cystometrogram) in PGE2 and SHR models of bladder dysfunction. SHR rats exhibited an increase in NVC frequency versus controls. Data is expressed as mean ± SE. N values as indicated. B - NVC frequency in individual PGE2 and SHR rats represented in A. Each PGE2 rat prior to PGE2 installation is used as its own control and each data point represents the mean for each experiment.

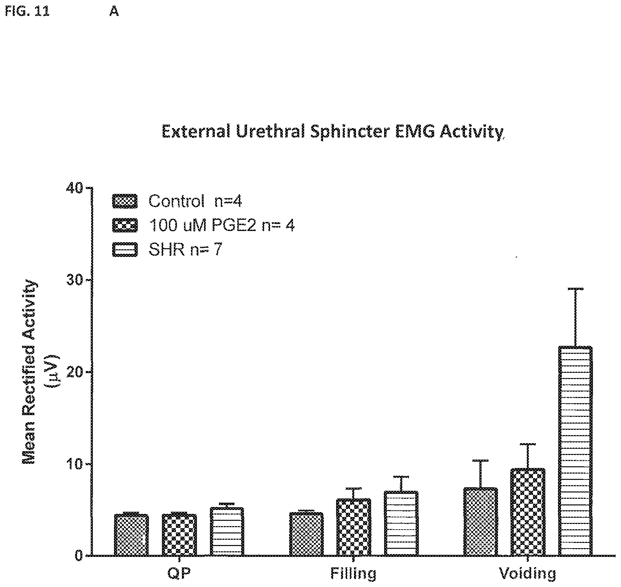

- Figure 11:: A - Mean external urethral sphincter (EUS) activity during the phases of a CMG event as measured by electromyography (EMG) in PGE2 and SHR models of bladder dysfunction (n values as indicated). Data is expressed as mean ± SE. PGE2 and SHR rats exhibit an increase in EUS EMG activity during the filling and voiding phases versus controls.

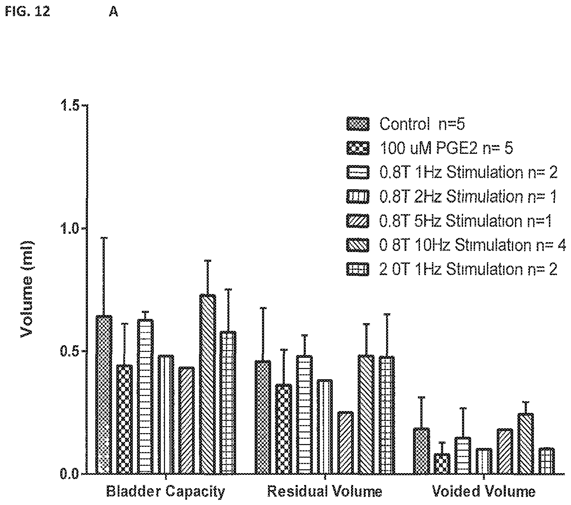

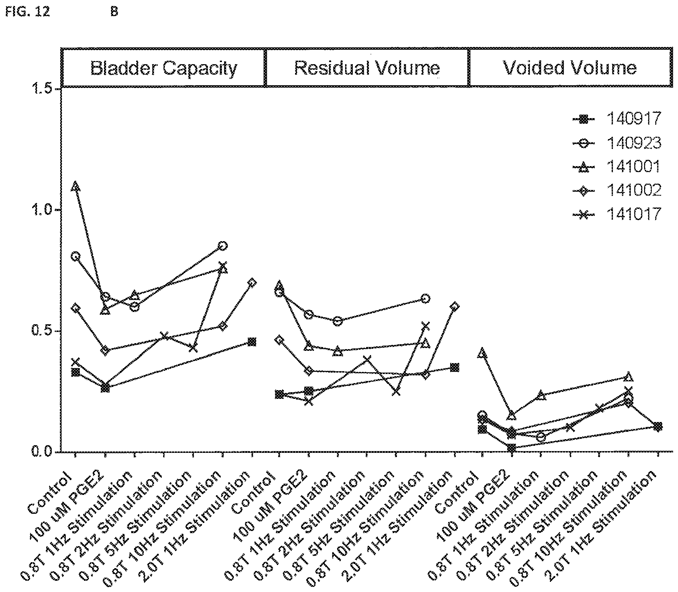

- Figure 12:: A - Pelvic nerve stimulation restores bladder capacity in PGE2 rats. The indicated electrical signals were applied to the pelvic nerves of PGE2 treated rats (n values as indicated). Data is expressed as mean ± SE. Stimulation of neural activity in the pelvic nerve as a result of the applied signal restores the bladder capacity. B - The data presented in A shown for individual rats to which the signals indicated were applied to the pelvic nerve. Each data point represents the mean for each experiment.

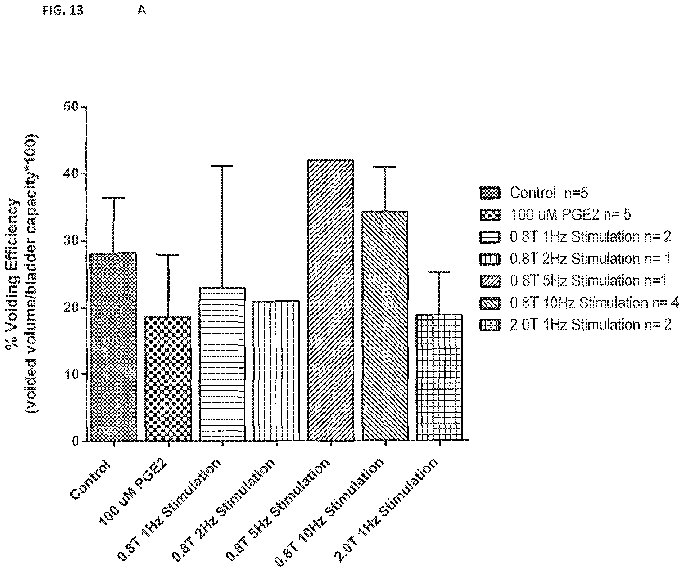

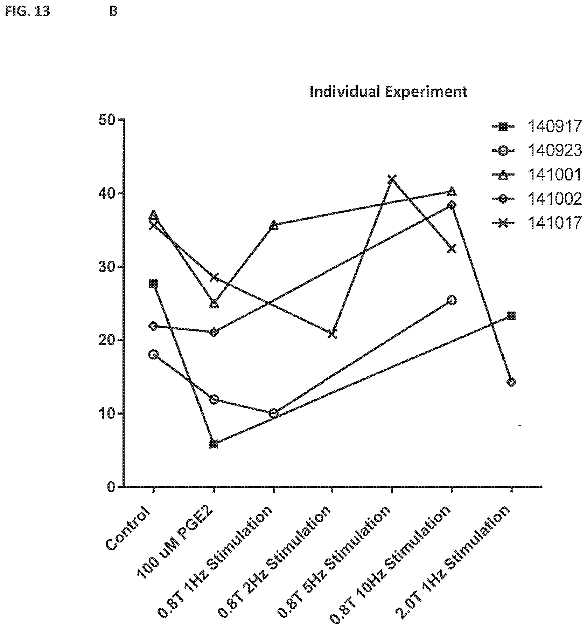

- Figure 13:: A - Pelvic nerve stimulation restores voiding efficiency in PGE2 rats. The indicated electrical signals were applied to the pelvic nerves of PGE2 treated rats (n values as indicated). Data is expressed as mean ± SE. Stimulation of neural activity in the pelvic nerve as a result of the applied signal restores the voiding efficiency. B - The data presented in A shown for individual rats to which the signals indicated were applied to the pelvic nerve. Each data point represents the mean for each experiment.

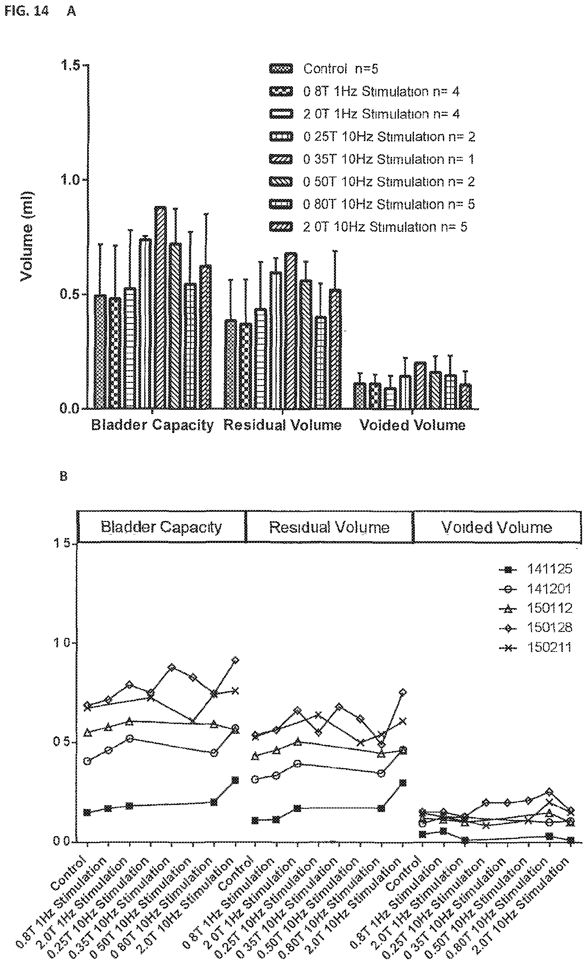

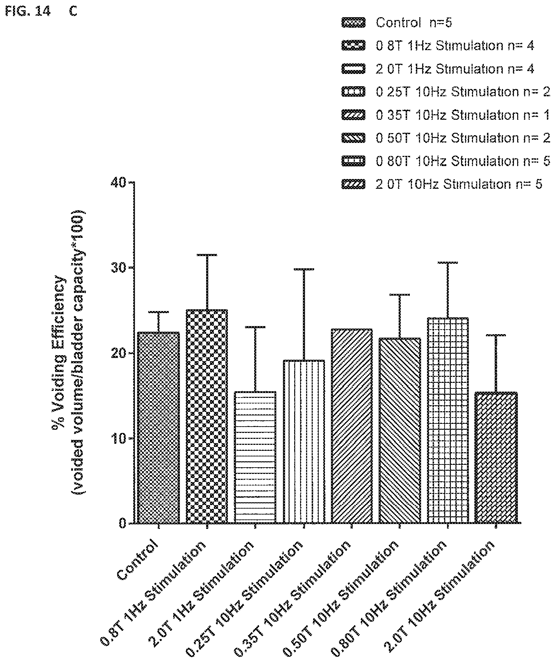

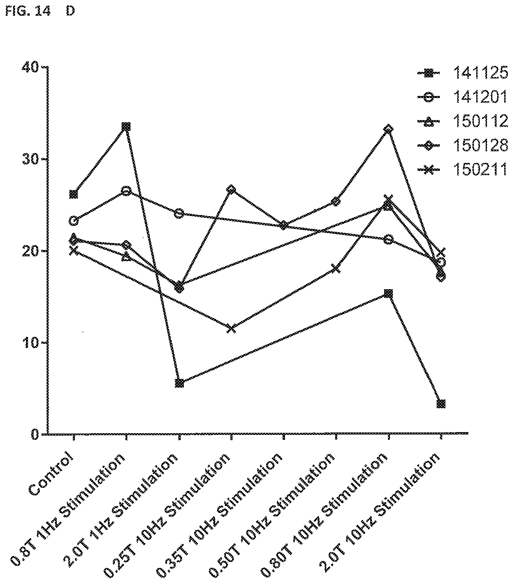

- Figure 14:: A - Pelvic nerve stimulation restores bladder capacity in SHR rats. Application of the indicated electrical signals to stimulate the pelvic nerve of SHR rats increased the bladder capacity compared to unstimulated controls. Data is expressed as mean ± SE. Stimulation of neural activity in the pelvic nerve as a result of the applied signal restores the bladder capacity. B - The data presented in A shown for individual rats to which the signals indicated were applied to the pelvic nerve. Each data point represents the mean for each experiment. C - Pelvic nerve stimulation restores voiding efficiency in SHR rats. Application of the indicated electrical signals to stimulate the pelvic nerve of SHR rats increased the voiding efficiency compared to unstimulated controls. Data is expressed as mean ± SE. Stimulation of neural activity in the pelvic nerve as a result of the applied signal restores the voiding efficiency. D - The data presented in C shown for individual rats to which the signals indicated were applied to the pelvic nerve. Each data point represents the mean for each experiment.

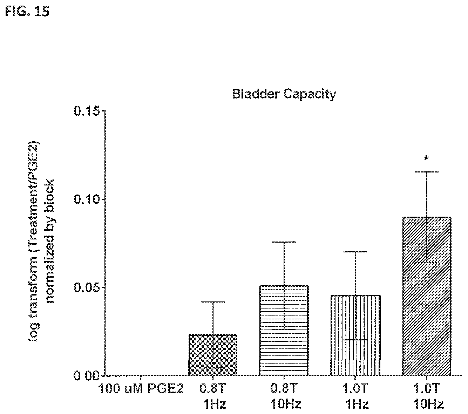

- Figure 15:: Mean bladder capacity pre- and post-stimulation of the pelvic nerve in PGE2 model of bladder dysfunction. PGE2 rats exhibited an increase in bladder capacity post stimulation versus PGE2 (no stimulation) condition. Each PGE2 rat prior to PGE2 installation is used as its own control. Data is expressed as mean ± SE. n = 11. * p < 0.05 vs 100 uM PGE2.

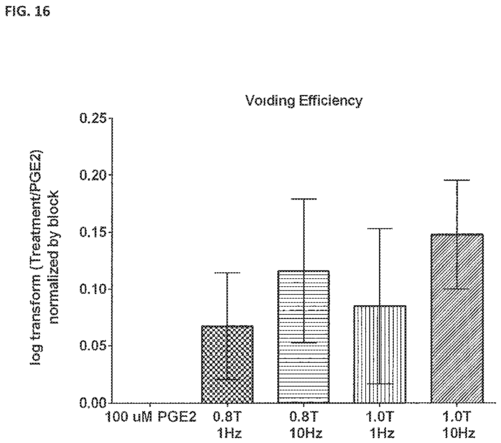

- Figure 16:: Voiding efficiency pre- and post-stimulation of the pelvic nerve in PGE2 model of bladder dysfunction. PGE2 rats exhibited an increase in voiding efficiency post stimulation versus PGE2 (no stimulation) condition. Data is expressed as mean ± SE. N values as indicated. N = 11. Each PGE2 rat prior to PGE2 installation is used as its own control.

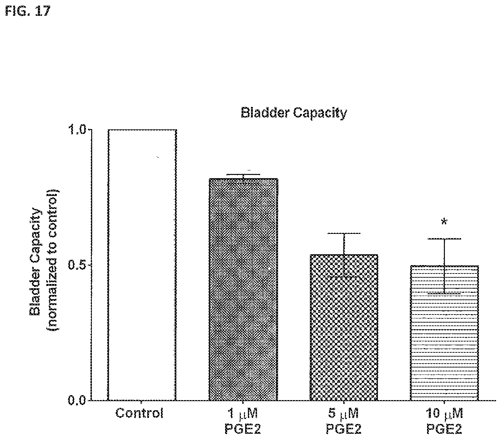

- Figure 17:: Mean bladder capacity in cat PGE2 model of bladder dysfunction. PGE2 cats exhibited a dose dependent reduction in bladder capacity versus controls. Data is expressed as mean ± SE. N values as indicated. Each PGE2 cat prior to PGE2 installation is used as its own control. N = 3. * p < 0.05 vs control.

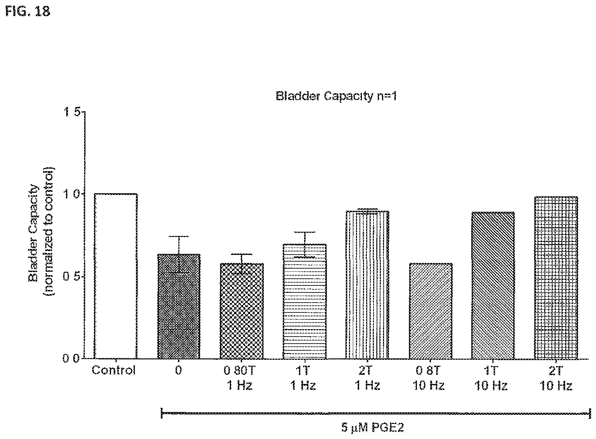

- Figure 18:: Mean bladder capacity pre- and post-stimulation of the pelvic nerve in cat PGE2 model of bladder dysfunction. Preliminary experiment in PGE2 cat shows a stimulation dependent increase in bladder capacity versus 5 µM PGE2. Data is expressed as mean of multiple experiments (in a single subject) ± SE. N =1.

The terms as used herein are given their conventional definition in the art as understood by the skilled person, unless otherwise defined below. In the case of any inconsistency or doubt, the definition as provided herein should take precedence.

As used herein, application of a signal may equate to the transfer of energy in a suitable form to carry out the intended effect of the signal. That is, application of a signal to a nerve or nerves may equate to the transfer of energy to (or from) the nerve(s) to carry out the intended effect. For example, the energy transferred may be electrical, mechanical (including acoustic, such as ultrasound), electromagnetic (e.g. optical), magnetic or thermal energy. It is noted that application of a signal as used herein does not include a pharmaceutical intervention.

As used herein, "actuator" is taken to mean any element of applying a signal to the nerve or plexus, for example an electrode, diode, Peltier element or ultrasound actuator.

As used herein, "neural activity" of a nerve is taken to mean the signalling activity of the nerve, for example the amplitude, frequency and/or pattern of action potentials in the nerve.

Modulation of neural activity, as used herein, is taken to mean that the signalling activity of the nerve is altered from the baseline neural activity - that is, the signalling activity of the nerve in the patient prior to any intervention. Such modulation may increase, inhibit, block, or otherwise change the neural activity compared to baseline activity.

Where the modulation of neural activity is an increase of neural activity, this may be an increase in the total signalling activity of the whole nerve, or that the total signalling activity of a subset of nerve fibres of the nerve is increased, compared to baseline neural activity in that part of the nerve. Preferably, the modulation of neural activity is an increase in the signalling activity of the afferent fibres of the nerve, optionally a selective increase in the signalling activity of the afferent fibres of the nerve. A selective increase in neural activity of the afferent fibres does not increase neural signalling in the efferent nerve fibres of the pelvic nerve, or alternatively, does not increase neural activity in the efferent nerve fibres of the pelvic nerve to a threshold level at which bladder pressure increases.

Where the modulation of neural activity is inhibition of neural activity, such inhibition may be partial inhibition. Partial inhibition may be such that the total signalling activity of the whole nerve is partially reduced, or that the total signalling activity of a subset of nerve fibres of the nerve is fully reduced (i.e. there is no neural activity in that subset of fibres of the nerve), or that the total signalling of a subset of nerve fibres of the nerve is partially reduced compared to baseline neural activity in that subset of fibres of the nerve. Where the modulation of neural activity is inhibition of neural activity, this also encompasses full inhibition of neural activity in the nerve - that is, there is no neural activity in the whole nerve.

Where inhibition of neural activity is a block on neural activity, such blocking may be a partial block - i.e. blocking of neural activity in a subset of nerve fibres of the nerve. Alternatively, such blocking may be a full block - i.e. blocking of neural activity in the whole nerve. A block on neural activity is understood to be blocking neural activity from continuing past the point of the block. That is, when the block is applied, action potentials may travel along the nerve or subset of nerve fibres to the point of the block, but not beyond the point of the block.

Modulation of neural activity may also be an alteration in the pattern of action potentials. It will be appreciated that the pattern of action potentials can be modulated without necessarily changing the overall frequency. For example, modulation of the neural activity may be such that the pattern of action potentials is altered to more closely resemble a healthy state rather than a disease state - *i.e.* to more closely resemble the pattern in a healthy individual.

Modulation of neural activity may comprise altering the neural activity in various other ways, for example increasing or inhibiting a particular part of the neural activity and/or stimulating new elements of activity, for example in particular intervals of time, in particular frequency bands, according to particular patterns and so forth. Such altering of neural activity may for example represent both increases and/or decreases with respect to the baseline activity.

Modulation of the neural activity may be temporary. As used herein, "temporary" is taken to mean that the modulated neural activity (whether that is an increase, inhibition, block or other modulation of neural activity or change in pattern versus baseline activity) is not permanent. That is, the neural activity following cessation of the signal is substantially the same as the neural activity prior to the signal being applied - i.e. prior to modulation.

Modulation of the neural activity may be persistent. As used herein, "persistent" is taken to mean that the modulated neural activity (whether that is an increase, inhibition, block or other modulation of neural activity or change in pattern versus baseline activity) has a prolonged effect. That is, upon cessation of the signal, neural activity in the nerve remains substantially the same as when the signal was being applied - i.e. the neural activity during and following modulation is substantially the same.

Modulation of the neural activity may be corrective. As used herein, "corrective" is taken to mean that the modulated neural activity (whether that is an increase, inhibition, block or other modulation of neural activity or change in pattern versus baseline activity) alters the neural activity towards the pattern of neural activity in a healthy individual. That is, upon cessation of the signal, neural activity in the nerve more closely resembles the pattern of action potentials in the nerve observed in a healthy subject than prior to modulation, preferably substantially fully resembles the pattern of action potentials in the nerve observed in a healthy subject.

Such corrective modulation caused by the signal can be any modulation as defined herein. For example, application of the signal may result in a block on neural activity, and upon cessation of the signal, the pattern of action potentials in the nerve resembles the pattern of action potentials observed in a healthy subject. By way of further example, application of the signal may result modulation such that the neural activity resembles the pattern of action potentials observed in a healthy subject, and upon cessation of the signal, the pattern of action potentials in the nerve resembles the pattern of action potentials observed in a healthy individual.

As used herein, a "healthy individual" or "healthy subject" is an individual not exhibiting any disruption or perturbation of normal bladder activity.

As used herein, "bladder dysfunction" is taken to mean that the patient or subject is exhibiting disruption of bladder function compared to a healthy individual. Bladder dysfunction may be characterised by symptoms such as nocturia, increased urinary retention, increased incontinence, increased urgency of urination or increased frequency of urination compared to a healthy individual. Bladder dysfunction includes conditions such as overactive bladder (OAB), neurogenic bladder, stress incontinence, and chronic urinary retention.

As used herein, an "improvement in a measurable physiological parameter" is taken to mean that for any given physiological parameter, an improvement is a change in the value of that parameter in the patient towards the normal value or normal range for that value - i.e. towards the expected value in a healthy individual.

For example, in a patient with bladder dysfunction, an improvement in a measurable parameter may be: a reduction in number of incontinence episodes, a decrease in urgency of urination, a decrease in frequency of urination, an increase in bladder capacity, an increase in bladder voiding efficiency, and/or a change in external urethral sphincter (EUS) activity towards that of a healthy individual, assuming the patient is exhibiting abnormal values for the respective parameter.

As used herein, a physiological parameter is not affected by modulation of the neural activity if the parameter does not change as a result of the modulation from the average value of that parameter exhibited by the subject or patient when no intervention has been performed - i.e. it does not depart from the baseline value for that parameter.

The skilled person will appreciate that the baseline for any neural activity or physiological parameter in an individual need not be a fixed or specific value, but rather can fluctuate within a normal range or may be an average value with associated error and confidence intervals. Suitable methods for determining baseline values would be well known to the skilled person.

As used herein, a measurable physiological parameter is detected in a patient when the value for that parameter exhibited by the patient at the time of detection is determined. In addition, the detection of the physiological parameter may include detection of a characteristic of the measured signal, for example amplitude or power, e.g., over a range of frequencies. A detector is any element able to make such a determination.

A "predefined threshold value" for a physiological parameter is the value for that parameter where that value or beyond must be exhibited by a subject or patient before the intervention is applied. For any given parameter, the threshold value may be defined as a value indicative of a pathological state (e.g. the patient is experiencing abnormal retention of urine) or a particular physiological state (e.g. the patient being asleep). Examples of such predefined threshold values include parasympathetic or sympathetic tone (neural, hemodynamic (e.g. heart rate, blood pressure, heart rate variability) or circulating plasma/urine biomarkers) greater than a threshold parasympathetic or sympathetic tone; abnormal bladder pressure compared to a healthy individual, abnormal bladder capacity compared to a healthy individual, bladder voiding efficiency lower than a healthy individual, abnormal pelvic nerve activity compared to a healthy individual (for instance a decrease in pelvic nerve activity), abnormal EUS activity compared to a healthy individual (for instance an increase in EUS activity), abnormal pudendal nerve activity (for instance a decrease in pudendal afferent activity), abnormal hypogastric nerve activity (for instance an increase in hypogastric nerve activity), or abnormal rate of change, e.g., increase in bladder pressure. Such a threshold value for a given physiological parameter is exceeded if the value exhibited by the patient is beyond the threshold value - that is, the exhibited value is a greater departure from the normal or healthy value for that parameter than the predefined threshold value.

The measurable physiological parameter may comprise an action potential or pattern of action potentials in one or more nerves of the patient, wherein the action potential or pattern of action potentials is associated with bladder dysfunction. Suitable nerves in which to detect an action potential or pattern of action potentials include a pelvic nerve, a pudendal nerve and/or a hypogastric nerve. In a particular example, the measurable physiological parameter comprises the pattern of action potentials in the pelvic nerve. The measureable physiological parameter may be muscle electromygraphic activity, wherein the electromyographic activity is indicative of the level of activity in the muscle. Such activity could typically be measured from the bladder detrusor muscle, the internal urethral sphincter, the external urethral sphincter, and the external anal sphincter. Treatment of bladder dysfunction, as used herein may be characterised by any one or more of a reduction in number of incontinence episodes, a decrease in urgency of urination, a decrease in frequency of urination, an increase bladder capacity, an increase in bladder voiding efficiency, a decrease in urinary retention, a change in external urethral sphincter (EUS) activity towards that of a healthy individual, and/or a change in the pattern of action potentials or activity of the pelvic nerve, pudendal nerve or hypogastric nerve towards that of a healthy individual.

Treatment of bladder dysfunction may be prophylactic or therapeutic.

A "neuromodulation apparatus" as used herein is an apparatus or device configured to modulate the neural activity of a nerve. Neuromodulation apparatuses or devices as described herein comprise at least one actuator capable of effectively applying a signal to a nerve. In those embodiments in which the neuromodulation apparatus is at least partially implanted in the patient, the elements of the apparatus that are to be implanted in the patient are constructed such that they are suitable for such implantation. Such suitable constructions would be well known to the skilled person. Indeed, various fully implantable neuromodulation devices have been implanted into human patients, such as the INTERSTIM™ devices of Medtronic, Inc (Minneapolis, MN), the Finetech-Brindley bladder control system (Finetech Medical, Hertfordshire, UK) and the BION™ devices of Advanced Bionics Corp.

As used herein, "implanted" is taken to mean positioned within the patient's body. Partial implantation means that only part of the apparatus is implanted - i.e. only part of the apparatus is positioned within the patient's body, with other elements of the apparatus external to the patient's body. Wholly implanted means that the entire apparatus is positioned within the patient's body.

For the avoidance of doubt, the apparatus being "wholly implanted" does not preclude additional elements, independent of the apparatus but in practice useful for its functioning (for example, a remote wireless charging unit or a remote wireless manual override unit), being independently formed and external to the patient's body.

As used herein, "charge-balanced" in relation to a DC current is taken to mean that the positive or negative charge introduced into any system (e.g. a nerve) as a result of a DC current being applied is balanced by the introduction of the opposite charge in order to achieve overall (i.e. net) neutrality. As used herein, a "pharmaceutical composition" is a composition suitable for administration to a subject or patient.

As used herein, a "compound for treating bladder dysfunction" is taken to mean a pharmacological compound capable of treating bladder dysfunction. Such compounds include antimuscarinic compounds, for example darifenacin, hyoscyamine, oxybutynin, tolterodine, solifenacin, trospium, or fesoterodine. Other examples are β-adrenergic receptor agonist compounds, optionally a β3-adrenergic receptor agonist, for example mirabegron. Another example is botulinum toxin.

### DETAILED DESCRIPTION

In accordance with the invention there is provided an apparatus for modulating the neural activity of the afferent fibres of at least one pelvic nerve of a patient as defined in claim 1, the apparatus comprising: a first actuator configured to apply a first signal to said at least one nerve; and a controller coupled to the first actuator and controlling the signal to be applied by the first actuator, such that the signal modulates the neural activity of the afferent fibres of the pelvic nerve to produce a physiological response in the patient.

Embodiments disclosed hereinafter which do not fall within the scope of the appended claims do not form part of the present invention.

In certain embodiments, the apparatus comprises a second actuator coupled to the controller and configured to apply a second signal to a pudendal nerve of the patient, wherein the controller controls the signal to be applied by the second actuator such that the signal modulates the neural activity of the afferent nerve fibres in the pudendal nerve to produce a physiological response in the patient.

In certain embodiments, the apparatus comprises one actuator configured to apply said first signal to only one of the left or right pelvic nerves of said patient. In this embodiment, the apparatus may further comprise a second actuator configured to apply said second signal to only one of the left or right pudendal nerves of said patient.

In certain embodiments, the modulation of the neural activity of the afferent fibres of the nerve or nerves is selective. A signal selectively modulates the neural activity of the afferent fibres of the pelvic nerve and/or pudendal nerve if that signal does not modulate the neural activity of the efferent fibres of those nerve(s), or if that signal modulates the neural activity of the efferent fibres of those nerves below a degree of modulation of the efferent fibres which leads to an increase in bladder pressure or voiding of the bladder (e.g. below the degree of modulation which leads to an increase in bladder pressure to the threshold pressure as defined in Andersson et al. Neurourology and Urodynamics, 30: 636-646 (2011)).

In certain embodiments, the signal applied by the one or more actuators is a non-destructive signal. As used herein, a "non-destructive signal" is a signal as defined above that, when applied, does not irreversibly damage the underlying neural signal conduction ability. That is, application of a non-destructive signal maintains the ability of the nerve or nerves (or fibres thereof) to conduct action potentials when application of the signal ceases, even if that conduction is in practice inhibited or blocked as a result of application of the non-destructive signal.

In those embodiments in which the apparatus has at least a first actuator and a second actuator, the signal which each of the actuators is configured to apply is independently selected from the signal to be applied by the other actuator.

In the passages below, the described embodiments of the signal apply to the first signal and, where applicable, may also apply to the second signal, independently of the first signal.

In certain embodiments, the signal which the first or second actuator is configured to apply is of a modality selected from an electrical signal, an optical signal, an ultrasonic signal, and a thermal signal. That is, each actuator may be configured to apply a different modality of signal. Alternatively, in certain embodiments each actuator is configured to apply the same modality of signal.

In certain embodiments, each actuator may be comprised of one or more electrodes, one or more photon sources, one or more ultrasound transducers, one more sources of heat, or one or more other types of actuator arranged to put the signal into effect.

In certain embodiments, the actuator is an electrode and the signal applied by the actuator is an electrical signal, for example a voltage or current. In certain such embodiments the signal applied comprises a direct current (DC) waveform, such as a charge balanced direct current waveform, or an alternating current (AC) waveform, or both a DC and an AC waveform.

In certain embodiments the signal comprises a sub-kilohertz frequency AC waveform. In certain such embodiments the signal comprises an AC waveform having a frequency of 0.1-500 Hz, preferably 0.1-50Hz, preferably 1-50Hz or 0.5-20 Hz, preferably 1-15Hz, for example 1, 2, 3, 4, 5, 6, 7, 8, 9, 10, 11, 12, 13, 14, 15 Hz, preferably 1-10Hz, for example 1, 2, 3, 4, 5, 6, 7, 8, 9, 10 Hz, preferably 1 or 10 Hz.

Typically, effective treatment of the symptoms of bladder dysfunction in accordance with the invention (for example overactive bladder (OAB), neurogenic bladder or other dysfunction of the lower urinary tract) requires the selection of appropriate stimulation parameters. Stimulation parameters include the stimulation pulse amplitude/intensity, the stimulation pulse duration, and stimulation frequency.

Relative stimulation pulse intensity can be expressed as multiples (0.1, 0.8, 1, 2, 5, etc.) of "T". "T" is the threshold stimulation intensity required to evoke a response a reflex electromyogram (EMG) response in the external urethral sphincter (EUS).

By way of example, T may be determined as follows: a low frequency electrical signal, typically 1 Hz, is applied and the intensity of stimulation is increased (either by increasing the voltage or the current of the signal, preferably the current) until the pelvic nerve stimulation pulse produces a reflex EMG response in the EUS. This stimulation intensity is designated T. The absolute threshold stimulation intensity may vary across individuals, and subsequent experimental or therapeutic intensities are designated as multiples of T to provide equivalent relative stimulation intensities.

The desired stimulation intensity (i.e. the desired multiple of threshold intensity "T") can be achieved through controlled variation of the current or voltage of the signal, preferably the current.

In certain embodiments the electrical signal has an amplitude value of from 0.1 T to 5.0 T, where T is a threshold obtained through empirical measurement of the threshold for the stimulation signal to evoke a reflex response in the external urethral sphincter or external anal sphincter, following application of stimulus to the pelvic nerve or pudendal nerve. In certain embodiments, the electrical signal has a T value of 0.1T-5.0T, 0.5T-2.5T or 0.2-3.0T, 0.25T-2.0T, for example 0.8T or 2.0T. In certain preferred embodiments the signal has a T value of 0.8T or 2.0T.

In certain preferred embodiments, the signal is an electrical signal comprising an AC waveform of 0.8T 1Hz, 0.8T 10Hz, 2.0T 1Hz, or 2.0T 10Hz.

In certain embodiments wherein the signal applied by the one or more actuators is a thermal signal, the signal reduces the temperature of the nerve (i.e. cools the nerve). In certain alternative embodiments, the signal increases the temperature of the nerve (i.e. heats the nerve). In certain embodiments, the signal both heats and cools the nerve.

In those embodiments in which the signal applied by the one or more actuators is a thermal signal, at least one of the one or more actuators is configured to apply a thermal signal. In certain such embodiments, all the actuators are configured to apply a thermal signal, optionally the same thermal signal.

In certain embodiments, one or more of the one or more actuators comprise a Peltier element configured to apply a thermal signal, optionally all of the one or more actuators comprise a Peltier element. In certain embodiments, one or more of the one or more actuators comprise a laser diode configured to apply a thermal signal, optionally all of the one or more actuators comprise a laser diode configured to apply a thermal signal (e.g. a diode configured to emit infrared radiation). In certain embodiments, one or more of the one or more actuators comprise an electrically resistive element configured to apply a thermal signal, optionally all of the one or more actuators comprise an electrically resistive element configured to apply a thermal signal.

In certain alternative embodiments, the signal applied by the one or more actuators is not a thermal signal.

In certain embodiments the signal applied by the one or more actuators is a mechanical signal, optionally an ultrasonic signal. In certain alternative embodiments, the mechanical signal applied by the one or more actuators is a pressure signal.

In certain embodiments the signal applied by the one or more actuators is an electromagnetic signal, optionally an optical signal. In certain such embodiments, the one or more actuators comprise a laser and/or a light emitting diode configured to apply the optical signal. In some embodiments, the apparatus further comprises a fibre optic interface configured to apply said signal from said one or more of the actuators to said at least one nerve.

It will be appreciated that in embodiments in which the apparatus comprises more than one actuator, the signal to be applied by each actuator is independently selected from the signal applied by the other actuator(s). For example, an apparatus according to the invention may comprise one actuator configured to apply a sub-kilohertz frequency AC waveform to the pelvic nerve in order to stimulate neural activity in the afferent fibres of the pelvic nerve, and a second actuator configured to apply a high frequency AC waveform to the pudendal nerve in order to inhibit or block signalling in the pudendal nerve. Alternatively, a first and a second actuator may be configured to stimulate neural activity in the pelvic nerve and in the pudendal nerve, respectively.

In certain embodiments, the physiological response produced in the patient is one or more of: a reduction in number of incontinence episodes, a reduction in the length and/or severity of incontinence episode(s), a decrease in urgency of urination, a decrease in frequency of urination, an increase bladder capacity, an increase in bladder voiding efficiency, a decrease in urinary retention and/or a change in external urethral sphincter (EUS) activity towards that of a healthy individual.

In certain embodiments, the apparatus further comprises one or more detector elements to detect one or more physiological parameters in the patient. Such a detector element may be configured to detect the one or more physiological parameters. That is, in such embodiments each detector may detect more than one physiological parameter, for example all the detected physiological parameters. Alternatively, in such embodiments each of the one or more detector elements is configured to detect a separate parameter of the one or more physiological parameters detected.

In certain embodiments, the one or more detected physiological parameters are selected from: parasympathetic tone, sympathetic tone, bladder pressure, bladder volume, external urethral sphincter activity, and the rate of change of any one of these parameters. In addition, the one or more detected physiological parameters may be selected from: nerve activity in the pelvic nerve, the hypogastric nerve or the pudendal nerve; muscle activity in the bladder detrusor muscle, the internal urethral sphincter, the external urethral sphincter or the external anal sphincter; and the rate of change of any one of these parameters. The skilled person will appreciate that the detection of the physiological parameter may include detection of the absolute value of that parameter, or a characteristic of the detection signal, for example amplitude or power, e.g., over a range of frequencies.

In an embodiment, the detector element is configured to detect nerve activity in the pelvic nerve (optionally, where the actuator is configured to apply a signal to one of the left or right pelvic nerve, the detector element is configured to detect nerve activity in the other of the left or right pelvic nerve). In another embodiment, the detector element is configured to detect nerve activity in the hypogastric nerve. In another embodiment, the detector element is configured to detect nerve activity in the pudendal nerve. In another embodiment, the detector element is configured to detect muscle activity in the bladder detrusor muscle. In another embodiment, the detector element is configured to detect muscle activity in the internal urethral sphincter. In another embodiment, the detector element is configured to detect muscle activity in the external urethral sphincter. In another embodiment, the detector element is configured to detect muscle activity in the external anal sphincter.

In such embodiments, the controller is coupled to the detector element configured to detect one or more physiological parameters, and causes the actuator or actuators to apply the signal when the physiological parameter is detected to be meeting or exceeding a predefined threshold value.

The inventors have observed, in an animal model of bladder dysfunction (in particular OAB), a decrease in pelvic nerve activity and an increase in hypogastric activity. Therefore, in certain embodiments, the one or more detected physiological parameters comprise an action potential or pattern of action potentials in one or more nerves of the patient, wherein the action potential or pattern of action potentials is associated with bladder dysfunction. In certain such embodiments, the one or more nerves are selected from a pelvic nerve, a pudendal nerve and a hypogastric nerve, preferably a pelvic nerve. In a preferred embodiment, the detected physiological parameter is a decrease in pelvic nerve activity, and/or an increase in hypogastric nerve activity.

It will be appreciated that any two or more of the indicated physiological parameters may be detected in parallel or consecutively. For example, in certain embodiments, the controller is coupled to a detector or detectors configured to detect the pattern of action potentials in the pelvic nerve at the same time as the bladder pressure in the patient.

In certain embodiments, the modulation in neural activity as a result of applying the signal is stimulation or an increase in neural activity in the nerve to which the signal is applied. That is, in such embodiments, application of the signal results in the neural activity in at least the afferent fibres of at least part of the nerve being stimulated or increased compared to the baseline neural activity in that part of the nerve. In certain embodiments, neural activity is increased across the whole nerve. In certain preferred embodiments, neural activity is selectively stimulated in the afferent fibres of the nerve to which the signal is applied (e.g. the pelvic nerve).

In certain embodiments, the modulation in neural activity as a result of applying the signal is an alteration to the pattern of action potentials in the nerve to which the signal is applied. In certain such embodiments, the neural activity is modulated such that the resultant pattern of action potentials in the nerve resembles the pattern of action potentials in the nerve or nerves observed in a healthy subject.

Modulation of neural activity may comprise altering the neural activity in various other ways, for example increasing or inhibiting a particular part of the activity and stimulating new elements of activity, for example in particular intervals of time, in particular frequency bands, according to particular patterns and so forth. Such altering of neural activity may for example represent both increases and/or decreases with respect to the baseline activity.

In certain embodiments, the controller causes the signal to be applied intermittently. In certain such embodiments, the controller causes the signal to applied for a first time period, then stopped for a second time period, then reapplied for a third time period, then stopped for a fourth time period. In such an embodiment, the first, second, third and fourth periods run sequentially and consecutively. The series of first, second, third and fourth periods amounts to one application cycle. In certain such embodiments, multiple application cycles can run consecutively such that the signal is applied in phases, between which phases no signal is applied. In certain embodiments, the signal applied for the first time period and the signal applied for the third time period are of the same parameters (frequency, amplitude, etc.) and the same modality. In other embodiments, the signal applied for the first and third time periods are of different parameters, and/or different modality.

In such embodiments, the duration of the first, second, third and fourth time periods is independently selected. That is, the duration of each time period may be the same or different to any of the other time periods. In certain such embodiments, the duration of each of the first, second, third and fourth time periods is any time from 5 seconds (5s) to 24 hours (24h), 30s to 12 h, 1 min to 12 h, 5 min to 8 h, 5 min to 6 h, 10 min to 6 h, 10 min to 4 h, 30 min to 4 h, 1 h to 4 h. In certain embodiments, the duration of each of the first, second, third and fourth time periods is 5s, 10s, 30s, 60s, 2 min, 5 min, 10 min, 20 min, 30 min, 40 min, 50 min, 60 min, 90 min, 2 h, 3 h, 4 h, 5 h, 6 h, 7 h, 8 h, 9 h, 10 h, 11 h, 12 h, 13 h, 14 h, 15 h, 16 h, 17 h, 18 h, 19 h, 20 h, 21 h, 22 h, 23 h, 24 h.

In certain embodiments wherein the controller causes the signal to be applied intermittently, the signal is applied for a specific amount of time per day. In certain such embodiments, the signal is applied for 10 min, 20 min, 30 min, 40 min, 50 min, 60 min, 90 min, 2 h, 3 h, 4 h, 5 h, 6 h, 7 h, 8 h, 9 h, 10 h, 11 h, 12 h, 13 h, 14 h, 15 h, 16 h, 17 h, 18 h, 19 h, 20 h, 21 h, 22 h, 23 h per day. In certain such embodiments, the signal is applied continuously for the specified amount of time. In certain alternative such embodiments, the signal may be applied discontinuously across the day, provided the total time of application amounts to the specified time.

In certain embodiments wherein the controller causes the signal to be applied intermittently, the signal is applied only when the patient is in a specific physiological state. In certain such embodiments, the signal is applied only when the patient exhibits a particular bladder pressure. In certain such embodiments, the signal is applied only when the patient is in a continent state. In certain such embodiments, the signal is applied only when the patient is in a state of bladder emptying. In certain such embodiments, the signal is applied only when the patient is asleep.

In certain such embodiments, the apparatus further comprises a communication, or input, element via which the status of the patient (e.g. that they about to go to sleep) can be indicated by the patient or a physician. In alternative embodiments, the apparatus further comprises a detector configured to detect the status of the patient, wherein the signal is applied only when the detector detects that the patient is in the specific state.

In certain embodiments of the apparatus, the modulation in neural activity caused by the application of the signal (whether that is an increase, inhibition, block or other modulation of neural activity) is temporary. That is, upon cessation of the signal, neural activity in the nerve or nerves returns substantially towards baseline neural activity within 1-60 seconds, or within 1-60 minutes, or within 1-24 hours, optionally 1-12 hours, optionally 1-6 hours, optionally 1-4 hours, optionally 1-2 hours. In certain such embodiments, the neural activity returns substantially fully to baseline neural activity. That is, the neural activity following cessation of the signal is substantially the same as the neural activity prior to the signal being applied - i.e. prior to modulation.

In certain alternative embodiments, the modulation in neural activity caused by the application of the signal or signals is substantially persistent. That is, upon cessation of the signal, neural activity in the nerve or nerves remains substantially the same as when the signal was being applied - i.e. the neural activity during and following modulation is substantially the same.

In certain embodiments, the modulation in neural activity caused by the application of the signal is partially corrective, preferably substantially corrective. That is, upon cessation of the signal, neural activity in the nerve or nerves more closely resembles the pattern of action potentials in the nerve(s) observed in a healthy subject than prior to modulation, preferably substantially fully resembles the pattern of action potentials in the nerve(s) observed in a healthy subject. In such embodiments, the modulation caused by the signal can be any modulation as defined herein. For example, application of the signal may result in a block on neural activity, and upon cessation of the signal, the pattern of action potentials in the nerve or nerves resembles the pattern of action potentials observed in a healthy individual. By way of further example, application of the signal may result in modulation such that the neural activity resembles the pattern of action potentials observed in a healthy subject, and upon cessation of the signal, the pattern of action potentials in the nerve or nerves resembles the pattern of action potentials observed in a healthy subject

In certain embodiments, the apparatus is suitable for at least partial implantation into the patient. In certain such embodiments, the apparatus is suitable to be fully implanted in the patient.

In certain embodiments, the apparatus further comprises one or more power supply elements, for example a battery, and/or one or more communication elements.

The present invention can be used in a method for treating bladder dysfunction in a patient, the method comprising implanting an apparatus as described above positioning the first actuator of the apparatus in signalling contact with a pelvic nerve of the patient, and activating the apparatus. In such embodiments, the actuator is in signalling contact with the nerve when it is positioned such that the signal can be effectively applied to the nerve. The apparatus is activated when the apparatus is in an operating state such that the signal will be applied as determined by the controller.

In certain examples, the actuator or actuators positioned in signalling contact with a pelvic nerve apply a signal to stimulate afferent neural activity in said pelvic nerve, preferably to selectively stimulate the afferent fibres of said pelvic nerve. In certain examples, the first actuator is positioned in signalling contact with only one of the left or right pelvic nerve. A detector element may be placed in signalling contact with the other of the left or right pelvic nerve.

In certain examples, the method comprises implanting an apparatus as described above, positioning the first actuator in signalling contact with a pelvic nerve of the patient to modulate the neural activity of said pelvic nerve, and positioning a second actuator in signalling contact with a pudendal nerve of the patient to modulate the neural activity of said pudendal nerve. In certain embodiments, the first actuator is positioned in signalling contact with only a pelvic nerve and the second actuator is positioned in signalling contact only with a pudendal nerve. In certain such embodiments the first actuator is configured to apply a signal to stimulate neural activity in said pelvic nerve, and the second actuator is configured to apply a signal to inhibit or block neural activity in said pudendal nerve. In certain alternative embodiments, the first actuator is configured to apply a signal to stimulate neural activity in said pelvic nerve, and the second actuator is configured to apply a signal to stimulate neural activity in said pudendal nerve.

In certain examples, the method is a method for treating overactive bladder or neurogenic bladder.

Implementation of the invention (as discussed both above and below) will be further appreciated by reference to Figures 2A-2C.

Figures 2A-2C show how the invention may be put into effect using one or more neuromodulation apparatuses which are implanted in, located on, or otherwise disposed with respect to a patient in order to carry out any of the various methods described herein. In this way, one or more neuromodulation apparatuses can be used to treat bladder dysfunction in a patient, by modulating neural activity in at least a pelvic nerve, optionally also a pudendal nerve.

In Figure 2A a separate neuromodulation apparatus 100 is provided for unilateral neuromodulation, although as discussed above and below a apparatus could be provided for bilateral neuromodulation (100, Fig. 2B and 2C). Each such neuromodulation apparatus may be fully or partially implanted in the patient, or otherwise located, so as to provide neuromodulation of the respective nerve or nerves. Figure 2A also schematically shows in the cutaway components of one of the neuromodulation apparatuses 100, in which the apparatus comprises several elements, components or functions grouped together in a single unit and implanted in the patient. A first such element is an actuator 102 which is shown in proximity to a pelvic nerve 90 of the patient. The apparatus may optionally further comprise further actuators (not shown) implanted proximally to a pudendal nerve. Alternatively, the pudendal nerve may be provided with a separate apparatus 100 (not shown). The actuator 102 may be operated by a controller element 104. The apparatus may comprise one or more further elements such as a communication element 106, a detector element 108, a power supply element 110 and so forth. Each neuromodulation apparatus 100 may operate independently, or may operate in communication with each other, for example using respective communication elements 106.

Each neuromodulation apparatus 100 may carry out the required neuromodulation independently, or in response to one or more control signals. Such a control signal may be provided by the controller 104 according to an algorithm, in response to output of one or more detector elements 108, and/or in response to communications from one or more external sources received using the communications element. As discussed herein, the detector element(s) could be responsive to a variety of different physiological parameters.

Figure 2B illustrates some ways in which the apparatus of Figure 2A may be differently distributed. For example, in Figure 2B the neuromodulation apparatuses 100 comprise actuators 102 implanted proximally to a pelvic nerve 90, optionally further comprising further actuators (not shown) implanted proximally to a pudendal nerve, but other elements such as a controller 104, a communication element 106 and a power supply 110 are implemented in a separate control unit 130 which may also be implanted in, or carried by the patient. The control unit 130 then controls the actuators in both of the neuromodulation apparatuses via connections 132 which may for example comprise electrical wires and/or optical fibres for delivering signals and/or power to the actuators.

In the arrangement of Figure 2B one or more detectors 108 are located separately from the control unit, although one or more such detectors could also or instead be located within the control unit 130 and/or in one or both of the neuromodulation apparatuses 100. The detectors may be used to detect one or more physiological parameters of the patient, and the controller element or control unit then causes the actuators to apply the signal in response to the detected parameter(s), for example only when a detected physiological parameter meets or exceeds a predefined threshold value. Physiological parameters which could be detected for such purposes include parasympathetic or sympathetic tone (neural, hemodynamic (e.g. heart rate, blood pressure, heart rate variability) or circulating plasma/urine biomarkers) greater than a threshold parasympathetic or sympathetic tone; abnormal bladder pressure compared to a healthy individual, abnormal bladder capacity compared to a healthy individual, bladder voiding efficiency lower than a healthy individual, abnormal pelvic nerve activity compared to a healthy individual (for instance a decrease in pelvic nerve activity), abnormal EUS activity compared to a healthy individual (for instance an increase in EUS activity), abnormal pudendal nerve activity (for instance a decrease in pudendal afferent activity), abnormal hypogastric nerve activity (for instance an increase in hypogastric nerve activity), or an abnormal rate of increasing bladder pressure. Physiological parameters which could be detected also include the power spectrum of the detected signal (for example via a fast fourier transform (FFT) or similar transform).

A variety of other ways in which the various functional elements could be located and grouped into the neuromodulation apparatuses, a control unit 130 and elsewhere are of course possible. For example, one or more sensors of Figure 2B could be used in the arrangement of Figures 2A or 2C or other arrangements.

Figure 2C illustrates some ways in which some functionality of the apparatus of Figures 2A or 2B is provided not implanted in the patient. For example, in Figure 2C an external power supply 140 is provided which can provide power to implanted elements of the apparatus in ways familiar to the skilled person, and an external controller 150 provides part or all of the functionality of the controller 104, and/or provides other aspects of control of the apparatus, and/or provides data readout from the apparatus, and/or provides a data input facility 152. The data input facility could be used by a patient or other operator in various ways, for example to input data relating to the activity status or bladder pressure.

Each neuromodulation apparatus may be adapted to carry out the neuromodulation required using one or more physical modes of operation which typically involve applying a signal to a pelvic nerve, optionally also to a pudendal nerve, such a signal or signals typically involving a transfer of energy to (or from) the nerve(s). As already discussed, such modes may comprise modulating the nerve or nerves using an electrical signal, an optical signal, an ultrasound or other mechanical signal, a thermal signal, a magnetic or electromagnetic signal, or some other use of energy to carry out the required modulation. Such signals may be non-destructive signals. Such modulation may comprise increasing, inhibiting, blocking or otherwise changing the pattern of neural activity in the nerve or nerves. Preferably the modulation comprises stimulating, optionally selectively stimulating, neural activity in the afferent fibres of the nerve or nerves. To this end, the actuator 90 illustrated in Figure 2A could be comprised of one or more electrodes, one or more photon sources, one or more ultrasound transducers, one more sources of heat, or one or more other types of actuators arranged to put the required neuromodulation into effect.

The neural modulation device(s) or apparatus may be arranged to stimulate (i.e. increase or induce) neural activity of a nerve, for example a pelvic nerve or a pudendal nerve, by using the actuator(s) to apply a voltage or current, for example a direct current (DC) such as a charge balanced direct current, or an AC waveform, or both. The device or apparatus may be arranged to use the actuator(s) to apply an AC waveform preferably 0.1-50Hz, preferably 1-50Hz or 0.5-20 Hz, preferably 1-15Hz, for example 1, 2, 3, 4, 5, 6, 7, 8, 9, 10, 11, 12, 13, 14, 15 Hz, preferably 1-10Hz, for example 1, 2, 3, 4, 5, 6, 7, 8, 9, 10 Hz, preferably 1 or 10 Hz.

In certain embodiments the AC waveform has an amplitude of from 0.5 T to 2.5 T, where "T" is the intensity of stimulation at which, for a given frequency (typically 1Hz), a reflex EMG response in the EUS is produced. The skilled person would be readily able to determine the appropriate value of T in any given patient.

In certain embodiments, the electrical signal has a T value of 0.1T-5.0T, 0.5T-2.5T, 0.2T-3.0T, 0.25T-2.0T, or 0.8T-2.0T, for example 0.8T, 0.9T, 1.0T, 1.1T, 1.2T, 1.3T, 1.4T, 1.5T, 1.6T, 1.8T, 1.9T, 2.0T, 2.5T, 3.0T. In certain preferred embodiments the signal has a T value of 0.8T or 2.0T, in particular 0.8T.

In certain preferred embodiments, the signal is an electrical signal comprising an AC waveform of 0.8T 1Hz, 0.8T 10Hz, 2.0T 1Hz, or 2.0T 10Hz.

The neural modulation device(s) or apparatus may be arranged to inhibit neural activity of a nerve, for example a pelvic nerve or a pudendal nerve, by using the actuator(s) to apply a voltage or current, for example a direct current (DC) such as a charge balanced direct current, or an AC waveform, or both. The device or apparatus may be arranged to use the actuator(s) to apply a DC ramp, then apply a first AC waveform, wherein the amplitude of the waveform increases during the period the waveform is applied, and then apply a second AC waveform. The AC waveform(s) may have a frequency of 5 to 50 KHz, optionally 5-10 KHz.

Thermal methods of neuromodulation typically manipulate the temperature of a nerve to inhibit signal propagation. For example, Patberg et al. (Blocking of impulse conduction in peripheral nerves by local cooling as a routine in animal experimentation. Journal of Neuroscience Methods 1984;10:267-75,) discuss how cooling a nerve blocks signal conduction without an onset response, the block being both reversible and fast acting, with onsets of up to tens of seconds. Heating the nerve can also be used to block conduction, and is generally easier to implement in a small implantable or localised actuator or device, for example using infrared radiation from laser diode or a thermal heat source such as an electrically resistive element, which can be used to provide a fast, reversible, and spatially very localised heating effect (see for example Duke et al. J Neural Eng. 2012 Jun;9(3):036003. Spatial and temporal variability in response to hybrid electro-optical stimulation). Either heating, or cooling, or both could be provided using a Peltier element.

Optogenetics is a technique that genetically modifies cells to express photosensitive features, which can then be activated with light to modulate cell function. Many different optogenetic tools have been developed that can be used to inhibit neural firing. A list of optogenetic tools to suppress neural activity has been compiled (Epilepsia. 2014 Oct 9. doi: 10.1111/epi.12804. WONOEP appraisal: Optogenetic tools to suppress seizures and explore the mechanisms of epileptogenesis. Ritter LM et al.). Acrylamine-azobenzene-quaternary ammonium (AAQ) is a photochromic ligand that blocks many types of K+channels and in the *cis* configuration, the relief of K+ channel block inhibits firing (Nat Neurosci. 2013 Jul;16(7):816-23. doi: 10.1038/nn.3424. Optogenetic pharmacology for control of native neuronal signaling proteins. Kramer RH et al.). By adapting Channelrhodopsin-2 and introducing it into mammalian neurons with the lentivirus, it is possible to control inhibitory synaptic transmission (Boyden ES 2005). Instead of using an external light source such as a laser or light emitting diode, light can be generated internally by introducing a gene based on firefly luciferase (Land BB 2014). The internally generated light has been sufficient to generate inhibition. Mechanical forms of neuromodulation can include the use of ultrasound which may conveniently be implemented using external instead of implanted ultrasound transducers. Other forms of mechanical neuromodulation include the use of pressure (for example see "The effects of compression upon conduction in myelinated axons of the isolated frog sciatic nerve" by Robert Fern and P. J. Harrison Br.j. Anaesth. (1975), 47, 1123).

Some electrical forms of neuromodulation may use direct current (DC), or alternating current (AC) waveforms applied to a nerve using one or more electrodes. A DC block may be accomplished by gradually ramping up the DC waveform amplitude (Bhadra and Kilgore, IEEE Transactions on Neural systems and rehabilitation engineering, 2004 12(3) pp. 313-324). Some AC techniques include HFAC or KHFAC (high-frequency or kilohertz frequency) to provide a reversible block (for example see Kilgore and Badra, 2004, Medical and Biological Engineering and Computing).

In the work of Kilgore and Bhadra, a proposed waveform was sinusoidal or rectangular at 3-5 kHz, and typical signal amplitudes that produced block were 3 - 5 Volts or 0.5 to 2.0 milli Amperes peak to peak.

HFAC may typically be applied at a frequency of between 1 and 50 kHz at a duty cycle of 100% (Bhadra, N. et al., Journal of Computational Neuroscience, 2007, 22(3), pp 313-326). Methods for selectively blocking activity of a nerve by application of a waveform having a frequency of 5 - 10 kHz are described in US 7,389,145. Similarly, US 8,731,676 describes a method of ameliorating sensory nerve pain by applying a 5-50 kHz frequency waveform to a nerve.

Some commercially available nerve blocking systems include the Maestro (RTM) system available from Enteromedics Inc. of Minnesota, USA. Similar neuromodulation devices are more generally discussed in US2014/214129 and elsewhere.

The techniques discussed above principally relate to the blocking of neuronal activity. Where modulation by increasing activity or otherwise modifying activity in various ways is required, electrodes adjacent to or in contact with the nerve or particular parts of the nerve for example in contact with specific nerve fibres may be used to impart an electrical signal to stimulate activity in various ways, as would be appreciated by the skilled person and as is described herein. By way of further example, devices for stimulating nerve activity in the pudendal nerve are described in US7571000 and US8396555.

The present invention can be used in a method of treating bladder dysfunction in a patient, the method comprising applying a first signal to at least one pelvic nerve of said patient to modulate the neural activity of said pelvic nerve in the patient. In certain embodiments, the method further comprises applying a second signal to a pudendal nerve of the patient to modulate the neural activity of said pudendal nerve.

In certain embodiments, the signal is applied by a neuromodulation apparatus comprising one or more actuators configured to apply the signal. In certain preferred embodiments the neuromodulation apparatus is at least partially implanted in the patient. In certain preferred embodiments, the neuromodulation apparatus is wholly implanted in the patient. For the avoidance of doubt, the apparatus being "wholly implanted" does not preclude additional elements, independent of the apparatus but in practice useful for its functioning (for example, a remote wireless charging unit or a remote wireless manual override unit), being independently formed and external to the patient's body.

In certain embodiments, the method is applied unilaterally. That is, in such embodiments the signal or signals are applied only to the left or only to the right pelvic nerve. In those embodiments in which a signal is also applied to a pudendal nerve, it may be applied to only to the corresponding pudendal nerve (i.e. a signal is applied to the left pelvic nerve and the left pudendal nerve, or the right pelvic nerve and the right pudendal nerve). In these embodiments, one or more detector element may be configured to detect nerve activity in the other of the left or right pelvic nerve (the nerve to which the signal is not applied). In certain alternative embodiments, the method is applied bilaterally. That is, in such embodiments, a signal is applied to the left and to the right pelvic nerve. In certain such embodiments wherein a signal is also applied to a pudendal nerve of the patient, such signal or signals may be applied bilaterally or unilaterally (i.e. to only the left or right, or to both pudendal nerves), preferably to both pudendal nerves.

In certain preferred embodiments, the modulation of neural activity as a result of applying the signal is an increase in neural activity.

In certain preferred embodiments, the signal or signals modulate (preferably stimulate) neural activity in the afferent fibres of the nerve to which the signal is applied. In certain preferred embodiments, the signal selectively modulates (preferably selectively stimulates) neural activity in the afferent fibres of the nerve to which the signal is applied (i.e. pelvic nerve and optionally the pudendal nerve). In certain preferred embodiments, the signal selectively stimulates neural activity in the afferent fibres of the pelvic nerve. A selective increase in neural activity of the afferent fibres does not increase neural signalling in the efferent nerve fibres of the pelvic nerve, or alternatively, does not increase neural activity in the efferent nerve fibres of the pelvic nerve to a threshold level at which bladder pressure increases.

In certain embodiments, the method is a method of treating overactive bladder. In certain embodiments, the method is a method of treating neurogenic bladder. In certain embodiments, the method is a method of treating nocturia. In certain embodiments, the method is a method of treating urinary incontinence. In certain embodiments, the method is a method of treating urine retention. It will be appreciated that the method may treat more than one of these conditions exhibited by a single patient - that is, the method may treat both nocturia and urine retention in the same patient.

In certain embodiments, the treatment of bladder dysfunction is prophylactic treatment. That is, the methods of the invention prevent the onset of bladder dysfunction. For example, the method may prevent or ameliorate the onset of bladder dysfunction in at risk patients, for example patients having diabetes, vitamin B12 deficiency, diseases of the central nervous system (e.g. brain tumours, multiple sclerosis, spina bifida), those in pregnancy or patients undergoing spinal surgery. Prophylactic treatment may also be such that it prevents an episode of bladder dysfunction. That is, in patients known to have bladder dysfunction, the methods may be used to prevent commencement of an episode of bladder dysfunction, for example by using the method to prevent onset of an episode of incontinence.

In certain embodiments, the treatment of bladder dysfunction is therapeutic treatment. That is, the methods of the invention at least partially restore the bladder function of the patient. For example, methods according to the invention may result in the patient exhibiting levels of urinary retention, incontinence, nocturia, urgency and/or frequency of urination closer to those levels of a healthy patient.

In certain embodiments of therapeutic methods, the methods may be interventional. That is, application of the method during an episode of incontinence, for example, results in the length and/or severity of the episode being reduced, or the episode stopped entirely.

In certain embodiments, treatment of bladder dysfunction is indicated by an improvement in a measurable physiological parameter, for example a reduction in number of incontinence episodes, a reduction in the length and/or severity of incontinence episode(s), a decrease in urgency of urination, a decrease in frequency of urination, an increase in bladder capacity, an increase in bladder voiding efficiency, a decrease in urinary retention, and/or a change in external urethral sphincter (EUS) activity towards that of a healthy individual.

Suitable methods for determining the value for any given parameter would be appreciated by the skilled person.

In certain embodiments, treatment of the condition is indicated by an improvement in the profile of neural activity in the nerve or nerves to which the signal is applied. That is, treatment of the condition is indicated by the neural activity in the nerve(s) approaching the neural activity in a healthy individual.

In those embodiments in which more than one signal may be applied (for example a first signal to a pelvic nerve and a second signal to a pudendal nerve, and/or the left and right signals when the method is applied bilaterally), each signal is selected independently of the others. As described below, the embodiments of the signal may apply to each such signal and are selected independently.

In certain embodiments, the modulation in neural activity as a result of applying the signal is an increase in neural activity in the nerve to which the signal is applied. That is, in such embodiments, application of the signal results in stimulation such that the neural activity in at least part of the nerve is increased compared to the baseline neural activity in that part of the nerve.

In certain preferred embodiments, the modulation in neural activity as a result of applying the signal is an increase in neural activity in the afferent fibres of the nerve or nerves. In certain preferred embodiments, the modulation in neural activity as a result of applying the signal is a selective increase in neural activity in the afferent fibres of the nerve or nerves. A signal that selectively increases neural activity in the afferent fibres does not stimulate neural activity in the efferent fibres of those nerve(s), or if that signal does stimulate neural activity in the efferent fibres of those nerves, it is below the degree of stimulation of the efferent fibres which leads to an increase in bladder pressure or voiding of the bladder (e.g. a degree of modulation which leads to an increase in bladder pressure to the threshold pressure as defined in Andersson et al. Neurourology and Urodynamics, 30: 636-646 (2011)).

In certain alternative embodiments, the modulation in neural activity as a result of applying the signal is an increase in neural activity in both the efferent and the afferent fibres of the nerve. In certain embodiments, a result of applying the signal is an increase in neural activity across the whole nerve. In certain embodiments, the neural activity in the efferent fibres may be increased but to a degree which is below the level of neural activity which leads to an increase in activity of the EUS.

It will be appreciated by the skilled person that stimulation of afferent neural activity may result in downstream reflex efferent neural activity. For the avoidance of doubt, such reflex efferent neural activity is not considered part of the modulation of neural activity as a result of the signal being applied - the modulation in neural activity is taken to be the activity directly caused by application of the signal, not any reflex response. For example, selective stimulation of afferent fibres of the pelvic nerve would not stimulate efferent neural activity directly (at least not to the extent necessary to increase bladder pressure and/or decrease urethral pressure, see above), but may result in subsequent efferent activity in the nerve due to a reflex response. It is within the ability of the skilled person to differentiate between direct neuromodulation as a result of the signal being applied and that induced by a reflex response.

In certain embodiments the modulation in neural activity as a result of applying the signal is inhibition of neural activity in the nerve to which the signal is applied. That is, in such embodiments, application of the signal results in the neural activity in at least part of the nerve being reduced compared to the baseline neural activity in that part of the nerve. In certain embodiments, the modulation in neural activity as a result of applying the signal is an inhibition in neural activity in the efferent fibres of the nerve. In certain embodiments, the modulation in neural activity as a result of applying the signal is an inhibition in neural activity in the afferent fibres of the nerve. In certain embodiments, the modulation in neural activity as a result of applying the signal is an inhibition in neural activity in both the efferent and the afferent fibres of the nerve.

In certain embodiments, the inhibition in neural activity as a result of applying the signal is a block on neural activity in the nerve to which the signal is applied. That is, in such embodiments, the application of the signal blocks action potentials from travelling beyond the point of the block in at least a part of the nerve. In certain such embodiments, the modulation is a partial block. In certain alternative embodiments, the modulation is a full block. In certain embodiments, the modulation in neural activity as a result of applying the signal is a block in neural activity in the efferent fibres of the nerve. In certain embodiments, the modulation in neural activity as a result of applying the signal is a block in neural activity in the afferent fibres of the nerve. In certain embodiments, the modulation in neural activity as a result of applying the signal is a block in neural activity in both the efferent and the afferent fibres of the nerve.

In certain embodiments, the modulation in neural activity as a result of applying the signal is an alteration to the pattern of action potentials in nerve to which the signal is applied. In certain such embodiments, the neural activity is modulated such that the resultant pattern of action potentials in the nerve resembles the pattern of action potentials in the nerve observed in a healthy subject.

In certain embodiments, the signal is applied intermittently. In certain such embodiments, the signal is applied for a first time period, then stopped for a second time period, then reapplied for a third time period, then stopped for a fourth time period. In such an embodiment, the first, second, third and fourth periods run sequentially and consecutively. The series of first, second, third and fourth periods amounts to one application cycle. In certain such embodiments, multiple application cycles can run consecutively such that the signal is applied in phases, between which phases no signal is applied.

In such embodiments, the duration of the first, second, third and fourth time periods is independently selected. That is, the duration of each time period may be the same or different to any of the other time periods. In certain such embodiments, the duration of each of the first, second, third and fourth time periods is any time from 5 seconds (5s) to 24 hours (24h), 30s to 12 h, 1 min to 12 h, 5 min to 8 h, 5 min to 6 h, 10 min to 6 h, 10 min to 4 h, 30 min to 4 h, 1 h to 4 h. In certain embodiments, the duration of each of the first, second, third and fourth time periods is 5s, 10s, 30s, 60s, 2 min, 5 min, 10 min, 20 min, 30 min, 40 min, 50 min, 60 min, 90 min, 2 h, 3 h, 4 h, 5 h, 6 h, 7 h, 8 h, 9 h, 10 h, 11 h, 12 h, 13 h, 14 h, 15 h, 16 h, 17 h, 18 h, 19 h, 20 h, 21 h, 22 h, 23 h, 24 h. In certain embodiments wherein the signal is applied intermittently, the signal is applied for a specific amount of time per day. In certain such embodiments, the signal is applied for 10 min, 20 min, 30 min, 40 min, 50 min, 60 min, 90 min, 2 h, 3 h, 4 h, 5 h, 6 h, 7 h, 8 h, 9 h, 10 h, 11 h, 12 h, 13 h, 14 h, 15 h, 16 h, 17 h, 18 h, 19 h, 20 h, 21 h, 22 h, 23 h per day. In certain such embodiments, the signal is applied continuously for the specified amount of time. In certain alternative such embodiments, the signal may be applied discontinuously across the day, provided the total time of application amounts to the specified time.

In certain embodiments wherein the signal is applied intermittently, the signal is applied only when the patient is in a specific state.. For example, the signal is applied only when the patient exhibits a particular bladder pressure, or when the patient is experiencing an episode of incontinence. In certain such embodiments, the signal is applied only when the patient is asleep.. In certain such embodiments, the signal is applied only when the patient is in a continent state. In certain such embodiments, the signal is applied only when the patient is in a state of bladder emptying. In such embodiments, the status of the patient (e.g. that they are about to go to sleep, or are experiencing an episode of incontinence) can be indicated by the patient. In alternative such embodiments, the status of the patient can be detected independently from any input from the patient. In certain embodiments in which the signal is applied by a neuromodulation apparatus, the apparatus further comprises a detector configured to detect the status of the patient, wherein the signal is applied only when the detector detects that the patient is in the specific state.

In certain embodiments of methods, the method further comprises the step of detecting one or more physiological parameters of the patient, wherein the signal is applied only when the detected physiological parameter meets or exceeds a predefined threshold value. In such embodiments wherein more than one physiological parameter is detected, the signal may be applied when any one of the detected parameters meets or exceeds its threshold value, alternatively only when all of the detected parameters meet or exceed their threshold values. In certain embodiments wherein the signal is applied by a neuromodulation apparatus, the apparatus further comprises at least one detector element configured to detect the one or more physiological parameters.

In certain embodiments, the one or more detected physiological parameters are one or more of: parasympathetic tone, sympathetic tone, bladder pressure, bladder volume, external urethral sphincter activity, and the rate of change of any one of these parameters. The measurable physiological parameter may comprise an action potential or pattern of action potentials in one or more nerves of the patient, wherein the action potential or pattern of action potentials is associated with bladder dysfunction. Suitable nerves in which to detect an action potential or pattern of action potentials include a pelvic nerve, a pudendal nerve and a hypogastric nerve. In a particular embodiment, the measurable physiological parameter comprises the pattern of action potentials in the pelvic nerve. The measureable physiological parameter may be muscle electromyographic activity or the rate of change of muscle electromyographic activity, wherein the electromyographic activity is indicative of the level of activity in the muscle, and such activity could be measured from the bladder detrusor muscle, the internal urethral sphincter, the external urethral sphincter, and the external anal sphincter.

In certain embodiments, the detected physiological parameter is an action potential or pattern of action potentials in one or more nerves of the patient, wherein the action potential or pattern of action potentials is associated with bladder dysfunction. In certain such embodiments, the nerve or nerves are selected from a pelvic nerve, a hypogastric nerve and/or a pudendal nerve. In certain such embodiments, the detected physiological parameter is a decrease in pelvic nerve activity and/or an increase in hypogastric nerve activity.

The skilled person will appreciate that the detection of the physiological parameter may include detection of the absolute value of that parameter, or a characteristic of the detection signal, for example amplitude or power, e.g., over a range of frequencies.

It will be appreciated that any two or more of the indicated physiological parameters may be detected in parallel or consecutively. For example, in certain embodiments, the pattern of action potentials in the pelvic nerve can be detected at the same time as bladder pressure.

In certain alternative embodiments, the signal is permanently applied. That is, once begun, the signal is continuously applied to the nerve or nerves. It will be appreciated that in embodiments wherein the signal is a series of pulses, gaps between pulses do not mean the signal is not continuously applied.

In certain embodiments of the methods, the modulation in neural activity caused by the application of the signal (whether that is an increase, inhibition, block or other modulation of neural activity) is temporary. That is, upon cessation of the signal, neural activity in the nerve or nerves returns substantially towards baseline neural activity within 1-60 seconds, or within 1-60 minutes, or within 1-24 hours, optionally 1-12 hours, optionally 1-6 hours, optionally 1-4 hours, optionally 1-2 hours. In certain such embodiments, the neural activity returns substantially fully to baseline neural activity. That is, the neural activity following cessation of the signal is substantially the same as the neural activity prior to the signal being applied - i.e. prior to modulation.

In certain alternative embodiments, the modulation in neural activity caused by the application of the signal is substantially persistent. That is, upon cessation of the signal, neural activity in the nerve or nerves remains substantially the same as when the signal was being applied - i.e. the neural activity during and following modulation is substantially the same.

In certain embodiments, the modulation in neural activity caused by the application of the signal is partially corrective, preferably substantially corrective. That is, upon cessation of the signal, neural activity in the nerve or nerves more closely resembles the pattern of action potentials observed in a healthy subject than prior to modulation, preferably substantially fully resembles the pattern of action potentials observed in a healthy subject. In such embodiments, the modulation caused by the signal can be any modulation as defined herein. For example, application of the signal may result in a block on neural activity, and upon cessation of the signal, the pattern of action potentials in the nerve or nerves resembles the pattern of action potentials observed in a healthy subject. By way of further example, application of the signal may result in modulation such that the neural activity resembles the pattern of action potentials observed in a healthy subject, and upon cessation of the signal, the pattern of action potentials in the nerve resembles the pattern of action potentials observed in a healthy subject. It is hypothesised that such a corrective effect is the result of a positive feedback loop.

In certain such embodiments, once first applied, the signal may be applied intermittently or permanently, as described in the embodiments above.

In certain embodiments wherein the modulation is bilateral, each signal is applied by a single neuromodulation apparatus. In certain alternative embodiments, the left-side signal(s) is applied by one neuromodulation apparatus and right-side signal(s) is applied by another neuromodulation apparatus.

In certain embodiments, the signal applied is a non-destructive signal.

In certain embodiments of the methods, the signal applied is an electrical signal, an electromagnetic signal (optionally an optical signal), a mechanical (optionally ultrasonic) signal, a thermal signal, a magnetic signal or any other type of signal.

In certain embodiments in which the signal is applied by a neuromodulation apparatus comprising at least one actuator, the actuator may be comprised of one or more electrodes, one or more photon sources, one or more ultrasound transducers, one more sources of heat, or one or more other types of actuator arranged to put the signal into effect.

In certain embodiments, the signal is an electrical signal, for example a voltage or current. In certain such embodiments the signal comprises a direct current (DC) waveform, such as a charge balanced DC waveform, or an alternating current (AC) waveform, or both a DC and an AC waveform. In those embodiments in which the signal is an electrical signal and is applied by a neuromodulation apparatus, the actuator is an electrode.

In certain embodiments, the signal is an AC waveform of 0.1-500 Hz, preferably 0.1-50Hz, preferably 1-50Hz or 0.5-20 Hz, preferably 1-15Hz, for example 1, 2, 3, 4, 5, 6, 7, 8, 9, 10, 11, 12, 13, 14, 15 Hz, preferably 1-10Hz, for example 1, 2, 3, 4, 5, 6, 7, 8, 9, 10 Hz, preferably 1 or 10 Hz. Such signals are particularly suitable for increasing or stimulating neural activity.

In certain embodiments the electrical signal has an intensity value of from 0.1 T to 5.0 T, where "T" is the intensity of stimulation at which, for a given frequency (typically 1Hz), a reflex EMG response in the EUS is produced.. In certain embodiments, the electrical signal has a T value of 0.1T-5.0T, 0.5T-2.5T, 0.2-3.0T, 0.25T-2.0T, or 0.8T-2.0T, for example 0.8T, 0.9T, 1.0T, 1.1T, 1.2T, 1.3T, 1.4T, 1.5T, 1.6T, 1.8T, 1.9T, 2.0T, 2.5T, 3.0T. In certain preferred embodiments the signal has a T value of 0.8T or 2.0T.

In certain preferred embodiments, the signal is an electrical signal comprising an AC waveform of 0.8T 1Hz, 0.8T 10Hz, 2.0T 1Hz, or 2.0T 10Hz.

In certain embodiments the signal comprises a DC ramp followed by a plateau and charge-balancing, followed by a first AC waveform, wherein the amplitude of the first AC waveform increases during the period in which the first AC waveform is applied, followed by a second AC waveform having a lower amplitude and/or lower frequency than the first AC waveform. In certain such embodiments, the DC ramp, first AC waveform and second AC waveform are applied substantially sequentially. In certain embodiments in which the signal comprises one or more AC waveforms, at least one of the AC waveforms has a frequency of 1 to 50 kHz, optionally 5 to 50 KHz, optionally 5-10 KHz. Such signals are particularly suitable for inhibiting or blocking neural activity.

In certain embodiments wherein the signal is a thermal signal, the signal reduces the temperature of the nerve (i.e. cools the nerve). In certain alternative embodiments, the signal increases the temperature of the nerve (i.e. heats the nerve). In certain embodiments, the signal both heats and cools the nerve.

In certain embodiments wherein the signal is a mechanical signal, the signal is an ultrasonic signal. In certain alternative embodiments, the mechanical signal is a pressure signal.

In certain examples, the present disclosure provides a method of treating bladder dysfunction (in particular overactive bladder), the method comprising applying a sub-kilohertz frequency AC electrical signal to a part or all of a pelvic nerve of said patient to increase the neural activity of said nerve, preferably in the afferent fibres of said nerve, preferably selectively increase neural activity in the afferent fibres of said nerve, wherein the signal is applied by a neuromodulation apparatus at least partially implanted in the patient. In certain embodiments, the method may further comprise applying a sub-kilohertz frequency AC electrical signal to a part or all of a pudendal nerve of the patient to stimulate neural activity in said pudendal nerve, preferably the afferent fibres of said pudendal nerve.

In certain such embodiments, the signal applied to the pudendal nerve may be applied by the same neuromodulation apparatus as applies the signal to the pelvic nerve, or alternatively by a second neuromodulation apparatus.

The present disclosure also relates to a neuromodulatory electrical waveform for use in treating bladder dysfunction in a patient, wherein the waveform is an AC waveform having a frequency of 0.5-20 Hz and intensity of 0.1T-5.0T, optionally 0.25-2.0T, optionally 0.8T-2.0T, such that, when applied to a pelvic nerve of the patient, the waveform selectively stimulates afferent neural signalling in the nerve. In certain embodiments, the waveform, when applied to the nerve, improves the patient's bladder function.

The present disclosure also relates to the use of a neuromodulation apparatus for treating bladder dysfunction in a patient by increasing neural activity in a pelvic nerve of the patient, preferably increasing the neural activity in the afferent fibres of said nerve, preferably selectively increasing the neural activity in the afferent fibres of said nerve.

The present disclosure also relates to a neuromodulation system, the system comprising a plurality of apparatuses as described above. In such a system, each apparatus may be arranged to communicate with at least one other apparatus, optionally all apparatuses in the system. In certain embodiments, the system is arranged such that, in use, the apparatuses are positioned to bilaterally modulate the neural activity of the afferent fibres of the pelvic nerves of a patient. In certain embodiments, the system is arranged such that, in use, the apparatuses are positioned to modulate the neural activity of the afferent fibres of at least one pelvic nerve of a patient and to modulate the activity of the afferent fibres of a pudendal nerve of the patient.

In such embodiments, the system may further comprise additional components arranged to communicate with the apparatuses of the system, for example a processor, a data input facility, a and/or a data display module. In certain such embodiments, the system further comprises a processor. In certain such embodiments, the processor is comprised within a mobile device (for example a smart phone) or computer.

The present disclosure also relates to a pharmaceutical composition comprising a compound for treating bladder dysfunction, for use in a method of treating bladder dysfunction in a subject, wherein the method is a method as described above, the method further comprising the step of administering an effective amount of the pharmaceutical composition to the subject. It is a preferred embodiment that the pharmaceutical composition is for use in a method of treating bladder dysfunction wherein the method comprises applying a first signal to a part or all of a pelvic nerve of said patient to stimulate the neural activity of said nerve in the patient, the signal being applied by a neuromodulation apparatus.

The present disclosure also relates to a pharmaceutical composition comprising a compound for treating bladder dysfunction, for use in treating bladder dysfunction in a subject, the subject having an apparatus as described above. That is, the pharmaceutical composition is for use in treating a subject that has had an apparatus as described above. The skilled person will appreciate that the apparatus has been implanted in a manner suitable for the apparatus to operate as described. Use of such a pharmaceutical composition in a patient having such an apparatus implanted will be particularly effective as it permits a cumulative or synergistic effect as a result of the combination of the compound for treating bladder dysfunction and apparatus operating in combination.

In certain examples, the compound for treating bladder dysfunction is selected from an antimuscarinic compound and a β-adrenergic receptor agonist, optionally a β3-adrenergic receptor agonist. In certain embodiments, the antimuscarinic compound is selected from darifenacin, hyoscyamine, oxybutynin, tolterodine, solifenacin, trospium, or fesoterodine. In certain embodiments, the β-adrenergic receptor agonist is a β3-adrenergic receptor agonist, for example mirabegron. In another embodiment, the pharmaceutical composition is botulinum toxin. In certain embodiments, the pharmaceutical composition is for use in treating OAB.

In certain embodiments, the pharmaceutical composition may comprise a pharmaceutical carrier and, dispersed therein, a therapeutically effective amount of the compounds for treating bladder dysfunction. The composition may be solid or liquid. The pharmaceutical carrier is generally chosen based on the type of administration being used and the pharmaceutical carrier may for example be solid or liquid. The compounds may be in the same phase or in a different phase than the pharmaceutical carrier.

Pharmaceutical compositions may be formulated according to their particular use and purpose by mixing, for example, excipient, binding agent, lubricant, disintegrating agent, coating material, emulsifier, suspending agent, solvent, stabilizer, absorption enhancer and / or ointment base. The composition may be suitable for oral, injectable or infusible (e.g., directly into the bladder), rectal or topical administration.

For example, the pharmaceutical composition may be administered orally, such as in the form of tablets, coated tablets, hard or soft gelatine capsules, solutions, emulsions, or suspensions. Administration can also be carried out rectally, for example using suppositories, locally or percutaneously, for example using ointments, creams, gels or solution, or parenterally, for example using injectable solutions.

For the preparation of tablets, coated tablets or hard gelatine capsules, the compounds for treating bladder dysfunction may be admixed with pharmaceutically inert, inorganic or organic excipients. Examples of suitable excipients include lactose, maize starch or derivatives thereof, talc or stearic acid or salts thereof. Suitable excipients for use with soft gelatine capsules include, for example, vegetable oils, waxes, fats and semi-solid or liquid polyols.

For the preparation of solutions and syrups, excipients include, for example, water, polyols, saccharose, invert sugar and glucose. For injectable solutions, excipients include, for example, water, alcohols, polyols, glycerine and vegetable oil. For suppositories and for local and percutaneous application, excipients include, for example, natural or hardened oils, waxes, fats and semi-solid or liquid polyols.

The pharmaceutical compositions may also contain preserving agents, solublizing agents, stabilizing agents, wetting agents, emulsifiers, sweeteners, colorants, odorants, buffers, coating agents and / or antioxidants.

Thus, a pharmaceutical formulation for oral administration may, for example, be granule, tablet, sugar coated tablet, capsule, pill, suspension or emulsion. For parenteral injection for, for example, intravenous, intramuscular or subcutaneous use, a sterile aqueous solution may be provided that may contain other substances including, for example, salts and / or glucose to make to solution isotonic. The compound may also be administered in the form of a suppository or pessary, or may be applied topically in the form of a lotion, solution, cream, ointment or dusting powder.

In a preferred embodiment of all aspects of the invention, the subject or patient is a mammal, more preferably a human. In certain embodiments, the subject or patient is suffering from bladder dysfunction.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims which define the scope of the present invention.

### Examples

### Example 1: Model validation

In the following examples, two accepted animal models of bladder dysfunction were used. The first was a PGE2 (prostoglandin E2) model, in which installation of PGE2 into rats induces a hyperactive bladder response. The second was a spontaneous hypertensive rat (SHR) model, in which the animals inherently exhibit abnormal bladder activity (compared to e.g. Wistar rats of their parent strain).

Animal models of bladder dysfunction need to take into account the fact that rodent (e.g. rat) urination behaviour differs from that of humans. However, the following parameters (as defined in Andersson et al. Neurourology and Urodynamics, 30: 636-646 published in 2011) are accepted measures against which therapeutic interventions can be assessed (adapted from Andersson *et al.*):
(a) Baseline pressure: minimum pressure between two micturitions. This parameter is important as an internal control for the procedure.
(b) Intermicturition pressure: mean pressure between two micturitions. The IMP is related to the occurrence of non-voiding contractions (NVCs). The more frequent and with higher amplitude the NVCs, the higher the IMP will be.
(c) Threshold pressure: intravesical pressure immediately before micturition.
(d) Maximum pressure: maximum bladder pressure during a micturition cycle. Also known as peak pressure, maximum voiding pressure, or maximum intravesical pressure. It should be noted that this pressure is not identical with the micturition pressure. The true micturition pressure is the pressure at which fluid starts to flow.
(e) Spontaneous activity (SA) or mean intermicturition oscillatory pressure: intermicturition pressure minus baseline pressure. This parameter is an approximate index of spontaneous bladder contractions between micturitions. SA will increase if there are frequent NVCs between micturitions.
(f) Non-voiding contractions (NVCs): defined as increases in intravesical pressure during cystometry, not associated with release of fluid. They are sometimes used as a surrogate for the involuntary contractions during filling that can be demonstrated in detrusor overactivity patients.
(g) Bladder capacity (BC): the volume held at micturation, calculated as voided volume + residual volume.
(h) Voided volume: volume voided in a given micturation (urination) event.
(i) Residual volume (RV): the volume remaining in the bladder after voiding.
(j) Delta (Δ) pressure: Threshold pressure minus baseline pressure.
(k) Bladder compliance: bladder capacity divided by Δ pressure. Since threshold pressure is the pressure at onset of micturition, subtraction of baseline pressure from threshold pressure gives the pressure variation between micturitions.
(l) Voiding efficiency: the voided volume expressed as a percentage of bladder capacity (i.e. voided volume/bladder capacity *100)
(m) External urethral sphincter electromyography: electrodes are placed into the external urethral sphincter and electromyography is recorded during bladder filling and voiding.

A number of the parameters above were measured for both PGE2 and SHR rats. These are shown in Figures 3-11.

### Methods:

### Surgical procedure:

A PE-90 polyethylene catheter was inserted into the bladder lumen through a small incision in the apex of the bladder dome. The bladder catheter was connected an infusion pump and a pressure transducer to measure bladder pressure. Additionally, two PFA-coated platinum-iridium wires (0.0055 inch diameter) were bilaterally inserted percutaneously into the external urethral sphincter (EUS) to record EUS EMG. Alternatively, a bipolar paddle electrode was placed intra-abdominally between the external urethral sphincter (EUS) and pubic symphysis (platinum-iridium contacts facing the urethra) to record EUS EMG.

### Cystometrograms (CMG's):

At the start of an experiment, the bladder was continuously filled with physiological saline at room temperature using an infusion pump with an open urethra for 45 minutes to allow recovery post-surgery. The bladder was subsequently emptied, and CMG's were recorded. A single CMG event was characterized by a quiet period, a filling period, and a voiding period.at the end of the voiding event, the infusion pump was turned off. The bladder was then emptied and subsequent CMG's were conducted.

### Animal Models:

PGE2: Control CMG's were conducted under saline infusion. After 2-3 subsequent control CMG events, the bladder was then infused continuously with PGE2 solution for 1 hour to allow for the bladder to reach drug related steady state. Multiple CMG's were then taken.

SHR: All CMG's were conducted under saline infusion.

### Measured Parameters

CMG parameters recorded were previously described by Andersson *et al.* as outlined above in example1: model validation.

Non-voiding contraction (NVC) parameters: during the filling phase for each CMG, non-voiding contractions (as described by Andersson *et al.*) were identified and defined as follows:
Bladder pressure area under the curve (AUC): it is the integral of the bladder pressure during a NVC.

NVC Duration: the time span between the start and end of a NVC event.

NVC Frequency: the number of NVC events during the filling phase of a CMG event.

These data show that PGE2 rats exhibit decreased voiding efficiency (i.e. they exhibit some urinary retention), exhibit a reduction in threshold and maximum bladder pressure, a reduction in Δpressure and a reduction in bladder capacity. SHR rats exhibited a reduction in threshold and maximum pressure, a reduction in Δpressure, and an increase in bladder compliance. In both models, these changes are indicative of an animal model of the symptoms of overactive bladder.

PGE2 and SHR rats also exhibited decreased magnitude and duration of NVCs, with SHR rats exhibiting an increased frequency of NVCs. Both PGE2 and SHR rats also exhibited abnormal external urethral sphincter (EUS) activity (Figure 11).

These figures show that these two models are characterised by disruption of at least one relevant parameter, making them suitable models for the assessment of the apparatuses of the invention.

### Example 2 - Therapeutic effect of neuromodulation

The PGE2 and SHR rates of the models validated in Example 1 underwent treatment in which the neural activity in the pelvic nerve was modulated by application of a signal to the nerve.

### Surgical isolation of the pelvic nerve:

The right pelvic (PeIN, central to the major pelvic ganglion) nerve was isolated and a bipolar stimulation electrode was placed (Teflon-coated Pt/lr wire, 10Ir7T). Prior to placing the PeIN onto the electrode, the abdominal skin was tied to a metal frame to create a bowl. The abdominal cavity was then filled with warm mineral oil. In some rat experiments, the right pelvic (PeIN, central to the major pelvic ganglion) nerve was isolated and a bipolar stimulation cuff electrode from CorTec (AirRay research Micro Cuff Tunnel 200µM with perpendicular leads, CorTec GmbH, Freiburg Germany). After surgery was completed, the abdomen was covered with cellophane to help maintain moisture and temperature.

### Stimulation and CMG's:

Stimulation of regulated current 100 µs per phase biphasic pulses were delivered across a range of frequencies (1- 30 Hz) and amplitudes (0.25- 2.0 times relative to the threshold to evoke reflex activity in the EUS EMG (Pelvic-EUS EMG). After control CMG's, in SHR and PGE2 CMG's in the PGE2 model, stimulation of the pelvic nerve was evaluated during subsequent CMG's. Stimulation was started at the onset of the filling phase and was subsequently turned off after the voiding event was completed. Voiding parameters described above were then compared to control.

Figure 12 shows that stimulation of a pelvic nerve in PGE2 rats restores the loss in bladder capacity back to the level in control rats. Signals of 0.8T 10Hz and 2.0T 1Hz were particularly effective.

Figure 13 shows that stimulation of the pelvic nerve was able to restore the voiding efficiency in rats given PGE2.

Figure 14 shows that in SHR rats, stimulation of the pelvic nerve resulted in an increase in bladder capacity (A), and in some instances was able to increase voiding efficiency (B - 0.8T 1Hz).

Further experimentation using larger sample sets supports the therapeutic effect of stimulating the pelvic nerve. Figure 15 echoes the data of Figure 12 and shows that stimulation of the pelvic nerve increases the bladder capacity of PGE2 rats.

Figure 16 again demonstrates that stimulation of the pelvic nerve is able to improve voiding efficiency in some instances. Efficacy is variable between individuals but Figure 16 demonstrates a trend towards increased voiding efficiency as a result of pelvic nerve stimulation.

### Cat model of OAB

Pelvic nerve stimulation is also effective in other models of OAB. Cats exhibit a urinary function similar to humans, and thus can represent a representative model for human disease. Administration of PGE2 to cats results in a dose dependent reduction in bladder capacity (Figure 17). However, stimulation of the pelvic nerve resulted in an increase in bladder capacity close to that observed in the control (Figure 18).

These data indicate that neuromodulation of the pelvic nerve is able to at least partially treat signs and symptoms of bladder dysfunction, and in certain cases fully restore bladder parameters to healthy levels. The data therefore shows that neuromodulation apparatuses according to the invention offer effective treatments for bladder dysfunction.

Based on the data presented herein, it is expected that co-modulation of a pudendal nerve of a subject together with modulation of the pelvic nerve would be advantageous in the treatment of bladder dysfunction. Modulation of the pudendal nerve to reduce detrusor over-activity has been described in US7571000, and based on the surprising effect of modulating neural activity of the pelvic nerve as described herein, the inventors expect co-modulation, for example stimulation, of the pudendal nerve together with the pelvic nerve would also be effective treatment for bladder dysfunction.

## Claims

1. An apparatus for modulating the neural activity of afferent fibres of at least one pelvic nerve of a patient, the apparatus comprising:
a first actuator configured to apply a first signal to said at least one pelvic nerve of the patient; and
a controller coupled to the first actuator, the controller controlling the signal to be applied by the actuator, such that the signal modulates the neural activity of the afferent fibres of the nerve to produce a physiological response in the patient,
wherein the first actuator is an electrode and the first signal is an electrical signal, and wherein the first signal comprises an AC waveform having a frequency of 1-10Hz and has a signal intensity of 0.25T-2.0T,
wherein "T" is the threshold stimulation intensity required to evoke a reflex electromyogram (EMG) response in the external urethral sphincter (EUS).

2. An apparatus according to claim 1, wherein the apparatus comprises a second actuator coupled to the controller and configured to apply a second signal to a pudendal nerve of the patient, wherein the controller controls the signal to be applied by the second actuator such that the signal modulates the neural activity of the pudendal nerve to produce a physiological response in the patient.

3. An apparatus according to claim 2, wherein the second signal is selected from an electrical signal, an optical signal, an ultrasonic signal and a thermal signal.

4. An apparatus according to claim 3, wherein the second signal is an electrical signal, and the one or more actuators configured to apply said electrical signal is an electrode.

5. An apparatus according to claim 4, wherein the second signal comprises an alternating current (AC) waveform.

6. An apparatus according to claim 4 or claim 5, wherein the first signal comprises a an AC waveform having a frequency of 1 Hz, 2 Hz, 3 Hz, 4Hz, 5 Hz, 6Hz, 7 Hz, 8 Hz, 9 Hz or 10 Hz.

7. An apparatus according to any one of claims 2-6, wherein the second signal comprises a sub-kilohertz frequency AC waveform, optionally an AC waveform having a frequency of 0.1-50 Hz, optionally 0.5-20 Hz, preferably 1-10Hz, for example 1 Hz, 2 Hz, 3 Hz, 4Hz, 5 Hz, 6Hz, 7 Hz, 8 Hz, 9 Hz or 10 Hz.

8. An apparatus according to any one of claims 4-7, wherein the first signal has a signal intensity of 0.8T-2.0T.

9. An apparatus according to any one of claims 4-8, wherein the second signal has a signal intensity of 0.1T-5.0T, optionally 0.2-3.0T, optionally 0.25-2.0T, optionally 0.8T-2.0T.

10. An apparatus according to any one of claims 1-9, wherein the physiological response is one or more of: a reduction in number of incontinence episodes, a reduction in the length and/or severity of incontinence episode(s), a decrease in urgency of urination, a decrease in frequency of urination, an increase bladder capacity, an increase in bladder voiding efficiency, a decrease in urinary retention, and/or a change in external urethral sphincter (EUS) activity towards that of a healthy individual.

11. An apparatus according to any one of claims 1-10, wherein the apparatus further comprises a detector element to detect one or more physiological parameters in the patient, wherein the controller is coupled to said detector element, and causes the first and/or second signal to be applied when the physiological parameter is detected to be meeting or exceeding a predefined threshold value,
optionally wherein the one or more of the detected physiological parameters is selected from sympathetic tone, parasympathetic tone, bladder pressure, bladder volume, external urethral sphincter activity, the rate of change of any one of said parameters, or comprises an action potential or pattern of action potentials in a nerve of the patient, wherein the action potential or pattern of action potentials is associated with bladder dysfunction, wherein the action potential or pattern of action potentials is in a pelvic nerve, a pudendal nerve or a hypogastric nerve.

12. An apparatus according to any one of claims 1-11, wherein application of the first signal increases neural activity in at least part of the pelvic nerve, optionally increases neural activity in the afferent fibres of the pelvic nerve, optionally selectively increases neural activity in the afferent fibres of the pelvic nerve.

13. An apparatus according to any one of claims 2-12, wherein application of the second signal increases neural activity in at least part of the pudendal nerve, optionally in the afferent fibres of the pudendal nerve.

14. An apparatus according to any one of claims 2-12, wherein application of the second signal inhibits neural activity in at least part of the pudendal nerve, optionally in the efferent or afferent fibres of the pudendal nerve, optionally across the whole nerve.

15. An apparatus according to any one of claims 1-14, wherein the modulation in neural activity as a result of the one or more actuators applying the signal is substantially persistent, or temporary or corrective.

## Patentansprüche

1. Vorrichtung zum Modulieren der neuronalen Aktivität von afferenten Fasern mindestens eines Beckennervs eines Patienten, wobei die Vorrichtung umfasst:
ein erstes Stellglied, das zum Anlegen eines erstes Signals an den mindestens einen Beckennerv des Patienten konfiguriert ist; und
eine mit dem ersten Stellglied gekoppelte Steuerung, wobei die Steuerung das von dem Stellglied anzulegende Signal so steuert, dass das Signal die neuronale Aktivität der afferenten Fasern des Nervs moduliert, um eine physiologische Reaktion in dem Patienten zu erzeugen,
wobei das erste Stellglied eine Elektrode ist und das erste Signal ein elektrisches Signal ist, und wobei das erste Signal eine AC-Wellenform mit einer Frequenz von 1-10 Hz umfasst und eine Signalintensität von 0,25 T-2,0 T aufweist,
wobei "T" die Schwellen-Stimulationsintensität ist, die erforderlich ist, um eine Reflex-Elektromyogramm (EMG)-Reaktion im äußeren Harnröhrenschließmuskel (EUS) hervorzurufen.

2. Vorrichtung nach Anspruch 1, wobei die Vorrichtung ein zweites Stellglied umfasst, das mit der Steuerung gekoppelt und zum Anlegen eines zweiten Signals an einen Nervus pudendus des Patienten konfiguriert ist, wobei die Steuerung das von dem zweiten Stellglied anzulegende Signal so steuert, dass das Signal die neuronale Aktivität des Nervus pudendus moduliert, um eine physiologische Reaktion bei dem Patienten zu erzeugen.

3. Vorrichtung nach Anspruch 2, wobei das zweite Signal ausgewählt ist aus einem elektrischen Signal, einem optischen Signal, einem Ultraschallsignal und einem thermischen Signal.

4. Vorrichtung nach Anspruch 3, wobei das zweite Signal ein elektrisches Signal ist und der eine oder die mehreren Stellglieder, die zum Anlegen des elektrischen Signals konfiguriert sind, eine Elektrode sind.

5. Vorrichtung nach Anspruch 4, wobei das zweite Signal eine Wechselstrom (AC)-Wellenform umfasst.

6. Vorrichtung nach Anspruch 4 oder Anspruch 5, wobei das erste Signal eine AC-Wellenform mit einer Frequenz von 1 Hz, 2 Hz, 3 Hz, 4Hz, 5 Hz, 6Hz, 7 Hz, 8 Hz, 9 Hz oder 10 Hz umfasst.

7. Vorrichtung nach einem der Ansprüche 2 bis 6, wobei das zweite Signal eine Subkilohertz-Frequenz-AC-Wellenform, gegebenenfalls eine AC-Wellenform mit einer Frequenz von 0,1 bis 50 Hz, gegebenenfalls 0,5 bis 20 Hz, vorzugsweise 1 bis 10 Hz, beispielsweise 1 Hz, 2 Hz, 3 Hz, 4 Hz, 5 Hz, 6 Hz, 7 Hz, 8 Hz, 9 Hz oder 10 Hz umfasst.

8. Vorrichtung nach einem der Ansprüche 4-7, wobei das erste Signal eine Signalintensität von 0,8 T-2,0 T aufweist.

9. Vorrichtung nach einem der Ansprüche 4-8, wobei das zweite Signal eine Signalintensität von 0,1 T-5,0 T, gegebenenfalls 0,2-3,0 T, gegebenenfalls 0,25-2,0 T, gegebenenfalls 0,8T-2,0 T aufweist.

10. Vorrichtung nach einem der Ansprüche 1 bis 9, wobei die physiologische Reaktion eine oder mehrere der folgenden ist: eine Verringerung der Anzahl der Inkontinenz-Episoden, eine Verringerung der Dauer und/oder des Schweregrads der Inkontinenz-Episode(n), eine Verringerung des Harndrangs, eine Verringerung der Häufigkeit des Wasserlassens, eine Erhöhung der Blasenkapazität, eine Erhöhung der Blasenentleerungseffizienz, eine Verringerung des Harnverhalts und/oder eine Änderung der Aktivität des äußeren Harnröhrenschließmuskels (EUS) in Richtung der Aktivität eines gesunden Individuums.

11. Vorrichtung nach einem der Ansprüche 1-10, wobei die Vorrichtung ferner ein Detektorelement zum Erfassen von einem oder mehreren physiologischen Parametern in dem Patienten umfasst, wobei die Steuerung mit dem Detektorelement gekoppelt ist und bewirkt, dass das erste und/oder zweite Signal angelegt wird, wenn erfasst wird, dass der physiologische Parameter einen vordefinierten Schwellenwert erreicht oder überschreitet,
wobei der eine oder mehrere der erfassten physiologischen Parameter ausgewählt sind aus Sympathikustonus, Parasympathikustonus, Blasendruck, Blasenvolumen, Aktivität des äußeren Harnröhrenschließmuskels, der Änderungsrate eines beliebigen der Parameter, oder ein Aktionspotential oder ein Muster von Aktionspotentialen in einem Nerv des Patienten umfasst, wobei das Aktionspotential oder das Muster von Aktionspotentialen mit einer Blasenfunktionsstörung assoziiert ist, wobei das Aktionspotential oder das Muster von Aktionspotentialen in einem Beckennerv, einem Nervus pudendus oder einem hypogastrischen Nerv vorhanden ist.

12. Vorrichtung nach einem der Ansprüche 1-11, wobei das Anlegen des ersten Signals die neuronale Aktivität in mindestens einem Teil des Beckennervs erhöht, gegebenenfalls die neuronale Aktivität in den afferenten Fasern des Beckennervs erhöht, gegebenenfalls die neuronale Aktivität in den afferenten Fasern des Beckennervs selektiv erhöht.

13. Vorrichtung nach einem der Ansprüche 2-12, wobei das Anlegen des zweiten Signals die neuronale Aktivität in mindestens einem Teil des Nervus pudendus, gegebenenfalls in den afferenten Fasern des Nervus pudendus erhöht.

14. Vorrichtung nach einem der Ansprüche 2-12, wobei das Anlegen des zweiten Signals die neuronale Aktivität in mindestens einem Teil des Nervus pudendus hemmt, gegebenenfalls in den efferenten oder afferenten Fasern des Nervus pudendus, gegebenenfalls über den gesamten Nerv.

15. Vorrichtung nach einem der Ansprüche 1-14, wobei die Modulation in der neuronalen Aktivität als Ergebnis des einen oder der mehreren Stellglieder, die das Signal anlegen, im Wesentlichen anhaltend oder vorübergehend oder korrigierend ist.

## Revendications

1. Appareil pour moduler l'activité neuronale des fibres afférentes d'au moins un nerf pelvien d'un patient, l'appareil comprenant :
un premier actionneur configuré pour appliquer un premier signal audit au moins un nerf pelvien du patient ; et
un dispositif de commande couplé au premier actionneur, le dispositif de commande commandant le signal à appliquer par l'actionneur, de sorte que le signal module l'activité neuronale des fibres afférentes du nerf pour produire une réponse physiologique chez le patient,
où le premier actionneur est une électrode et le premier signal est un signal électrique, et où le premier signal comprend une forme d'onde de courant alternatif ayant une fréquence de 1 à 10 Hz et a une intensité de signal de 0,25 T à 2,0 T,
où "T" est l'intensité de stimulation seuil requise pour provoquer une réponse d'électromyogramme (EMG) réflexe dans le sphincter urétral externe (EUS).

2. Appareil selon la revendication 1, où l'appareil comprend un deuxième actionneur couplé au dispositif de commande et configuré pour appliquer un deuxième signal à un nerf pudendal du patient, où le dispositif de commande commande le signal à appliquer par le deuxième actionneur de sorte que le signal module l'activité neuronale du nerf pudendal pour produire une réponse physiologique chez le patient.

3. Appareil selon la revendication 2, dans lequel le deuxième signal est sélectionné parmi un signal électrique, un signal optique, un signal ultrasonore et un signal thermique.

4. Appareil selon la revendication 3, dans lequel le deuxième signal est un signal électrique, et les un ou plusieurs actionneurs configurés pour appliquer ledit signal électrique sont une électrode.

5. Appareil selon la revendication 4, dans lequel le deuxième signal comprend une forme d'onde de courant alternatif (AC).

6. Appareil selon la revendication 4 ou la revendication 5, dans lequel le premier signal comprend une forme d'onde de courant alternatif ayant une fréquence de 1 Hz, 2 Hz, 3 Hz, 4 Hz, 5 Hz, 6 Hz, 7 Hz, 8 Hz, 9 Hz ou 10 Hz.

7. Appareil selon l'une quelconque des revendications 2 à 6, dans lequel le deuxième signal comprend une forme d'onde de courant alternatif de fréquence inférieure au kilohertz, facultativement une forme d'onde de courant alternatif ayant une fréquence de 0,1 à 50 Hz, facultativement de 0,5 à 20 Hz, de préférence de 1 à 10 Hz, par exemple 1 Hz, 2 Hz, 3 Hz, 4 Hz, 5 Hz, 6 Hz, 7 Hz, 8 Hz, 9 Hz ou 10 Hz.

8. Appareil selon l'une quelconque des revendications 4 à 7, dans lequel le premier signal a une intensité de signal de 0,8 T à 2,0 T.

9. Appareil selon l'une quelconque des revendications 4 à 8, dans lequel le deuxième signal a une intensité de signal de 0,1 T à 5,0 T, facultativement de 0,2 à 3,0 T, facultativement de 0,25 à 2,0 T, facultativement de 0,8 T à 2,0 T.

10. Appareil selon l'une quelconque des revendications 1 à 9, dans lequel la réponse physiologique est une ou plusieurs parmi : une réduction du nombre d'épisodes d'incontinence, une réduction de la durée et/ou de la gravité d'épisodes d'incontinence, une diminution de l'urgence de la miction, une diminution de la fréquence de la miction, une augmentation de la capacité de la vessie, une augmentation de l'efficacité de l'évacuation de la vessie, une diminution de la rétention urinaire, et/ou un changement de l'activité de sphincter urétral externe (EUS) par rapport à celle d'un individu sain.

11. Appareil selon l'une quelconque des revendications 1 à 10, où l'appareil comprend en outre un élément détecteur pour détecter un ou plusieurs paramètres physiologiques chez le patient, où le dispositif de commande est couplé audit élément détecteur, et amène le premier et/ou le deuxième signal à être appliqués lorsque le paramètre physiologique est détecté comme atteignant ou dépassant une valeur seuil prédéfinie,
facultativement, où les un ou plusieurs paramètres physiologiques détectés sont choisis parmi le tonus sympathique, le tonus parasympathique, la pression de la vessie, le volume de la vessie, l'activité de sphincter urétral externe, le taux de changement de l'un quelconque desdits paramètres, ou comprennent un potentiel d'action ou un schéma de potentiels d'action dans un nerf du patient, où le potentiel d'action ou le schéma de potentiels d'action est associé à un dysfonctionnement de la vessie, où le potentiel d'action ou le schéma de potentiels d'action se trouve dans un nerf pelvien, un nerf pudendal ou un nerf hypogastrique.

12. Appareil selon l'une quelconque des revendications 1 à 11, dans lequel l'application du premier signal augmente l'activité neuronale dans au moins une partie du nerf pelvien, facultativement augmente l'activité neuronale dans les fibres afférentes du nerf pelvien, facultativement augmente sélectivement l'activité neuronale dans les fibres afférentes du nerf pelvien.

13. Appareil selon l'une quelconque des revendications 2 à 12, dans lequel l'application du deuxième signal augmente l'activité neuronale dans au moins une partie du nerf pudendal, facultativement dans les fibres afférentes du nerf pudendal.

14. Appareil selon l'une quelconque des revendications 2 à 12, dans lequel l'application du deuxième signal inhibe l'activité neuronale dans au moins une partie du nerf pudendal, facultativement dans les fibres efférentes ou afférentes du nerf pudendal, facultativement à travers le nerf entier.

15. Appareil selon l'une quelconque des revendications 1 à 14, dans lequel la modulation de l'activité neuronale en raison de l'application du signal par les un ou plusieurs actionneurs est essentiellement persistante, temporaire ou corrective.
